# EUROPEAN PATENT SPECIFICATION

(11) **EP 2 998 008 B1**
(45) Date of publication and mention of the grant of the patent: **07.02.2018**
(21) Application number: 14185060.2
(22) Date of filing: 17.09.2014
(51) Int. Cl.: B01D 15/08, C07K 14/46

(54) **Product protective capture and elution of N-glycosylated therapeutic glycoproteins and enveloped viruses**
Schützende Produkterfassung und Elution von therapeutischen N-glycosylierten Glycoproteinen und umhüllten Viren
Protection de produit de saisie et d'élution de glycoprotéines thérapeutiques n-glycosylées et de virus enveloppés

(43) Date of publication of application: 23.03.2016
(73) Proprietor: Hochschule für Technik und Wirtschaft (HTW) Berlin, 10318 Berlin (DE)
(72) Inventor: von Horsten, Hans Henning, 10245 Berlin (DE)
(74) Representative: Geling, Andrea

(56) References cited:
- WO-A1-01/85296
- HENGYE LI ET AL: "A Wulff-type boronate for boronate affinity capture of cis-diol compounds at medium acidic pH condition", CHEMICAL COMMUNICATIONS, vol. 47, no. 28, 1 January 2011 (2011-01-01), page 8169, XP055174295, ISSN: 1359-7345, DOI: 10.1039/c1cc11096a
- HENGYE LI ET AL: "A benzoboroxole-functionalized monolithic column for the selective enrichment and separation of cis-diol containing biomolecules", CHEMICAL COMMUNICATIONS, vol. 48, no. 34, 1 January 2012 (2012-01-01), page 4115, XP055174296, ISSN: 1359-7345, DOI: 10.1039/c2cc30230f
- CALEB BROMBA ET AL: "Phenyl boronic acid complexes of diols and hydroxyacids", SUPRAMOLECULAR CHEMISTRY, vol. 21, no. 1-2, 1 January 2009 (2009-01-01), pages 81-88, XP055174459, ISSN: 1061-0278, DOI: 10.1080/10610270802527044
- KNAPPSKOG ET AL: "The level of synthesis and secretion of Gaussia princeps luciferase in transfected CHO cells is heavily dependent on the choice of signal peptide", JOURNAL OF BIOTECHNOLOGY, ELSEVIER SCIENCE PUBLISHERS, AMSTERDAM, NL, vol. 128, no. 4, 6 March 2007 (2007-03-06) , pages 705-715, XP005916232, ISSN: 0168-1656, DOI: 10.1016/J.JBIOTEC.2006.11.026
- INN H. YUK ET AL: "Effects of copper on CHO cells: Insights from gene expression analyses", BIOTECHNOLOGY PROGRESS., vol. 30, no. 2, 21 January 2014 (2014-01-21), pages 429-442, XP055309092, US ISSN: 8756-7938, DOI: 10.1002/btpr.1868
- FRANCESCA ZAGARI ET AL: "Lactate metabolism shift in CHO cell culture: the role of mitochondrial oxidative activity", NEW BIOTECHNOLOGY, vol. 30, no. 2, 1 January 2013 (2013-01-01), pages 238-245, XP055295213, NL ISSN: 1871-6784, DOI: 10.1016/j.nbt.2012.05.021
- HUONG LE ET AL: "Multivariate analysis of cell culture bioprocess data-Lactate consumption as process indicator", JOURNAL OF BIOTECHNOLOGY, vol. 162, no. 2-3, 1 December 2012 (2012-12-01), pages 210-223, XP055287323, AMSTERDAM, NL ISSN: 0168-1656, DOI: 10.1016/j.jbiotec.2012.08.021
- BHANU CHANDRA MULUKUTLA ET AL: "On metabolic shift to lactate consumption in fed-batch culture of mammalian cells", METABOLIC ENGINEERING, ACADEMIC PRESS, US, vol. 14, no. 2, 16 December 2011 (2011-12-16), pages 138-149, XP028466094, ISSN: 1096-7176, DOI: 10.1016/J.YMBEN.2011.12.006 [retrieved on 2012-01-08]
- H.J CRUZ ET AL: "Effects of ammonia and lactate on growth, metabolism, and productivity of BHK cells", ENZYME AND MICROBIAL TECHNOLOGY, vol. 27, no. 1-2, 1 July 2000 (2000-07-01) , pages 43-52, XP055349769, US ISSN: 0141-0229, DOI: 10.1016/S0141-0229(00)00151-4
- LISTE-CALLEJA LETICIA ET AL: "Lactate and glucose concomitant consumption as a self-regulated pH detoxification mechanism inHEK293cell cultures", APPLIED MICROBIOLOGY AND BIOTECHNOLOGY, SPRINGER, DE, vol. 99, no. 23, 14 August 2015 (2015-08-14), pages 9951-9960, XP035870267, ISSN: 0175-7598, DOI: 10.1007/S00253-015-6855-Z [retrieved on 2015-08-14]

### SEQUENCE LISTING

<110> HTW Berlin
<120> PRODUCT PROTECTIVE CAPTURE AND ELUTION OF N-GLYCOSYLATED THERAPEUTIC GLYCOPROTEINS AND ENVELOPED VIRUSES
<130> 886-1 EP
<160> 3
<170> PatentIn version 3.5
<210> 1
   <211> 394
   <212> PRT
   <213> Mycobacterium smegmatis
<400> 1
   Met Ser Asn Trp Gly Asp Tyr Glu Asn Glu Ile Tyr Gly Gln Gly Leu
   1               5                   10                  15
   Val Gly Val Ala Pro Thr Leu Pro Met Ser Tyr Ala Asp Trp Glu Ala
               20                  25                  30
   His Ala Gln Gln Ala Leu Pro Pro Gly Val Leu Ser Tyr Val Ala Gly
           35                  40                  45
   Gly Ser Gly Asp Glu His Thr Gln Arg Ala Asn Val Glu Ala Phe Lys
       50                  55                  60
   His Trp Gly Leu Met Pro Arg Met Leu Met Ala Ala Thr Glu Arg Asp
   65                  70                  75                  80
   Leu Ser Val Glu Leu Trp Gly Lys Thr Trp Ala Ala Pro Met Phe Phe
                   85                  90                  95
   Ala Pro Ile Gly Val Ile Ala Leu Cys Ala Gln Asp Gly His Gly Asp
               100                 105                 110
   Ala Ala Ser Ala Gln Ala Ser Ala Arg Thr Gly Val Pro Tyr Ile Thr
           115                 120                 125
   Ser Thr Leu Ala Val Ser Ser Leu Glu Asp Ile Arg Lys His Ala Gly
       130                 135                 140
   Asp Thr Pro Ala Tyr Phe Gln Leu Tyr Tyr Pro Glu Asp Arg Asp Leu
   145                 150                 155                 160
   Ala Glu Ser Phe Ile Arg Arg Ala Glu Glu Ala Gly Tyr Asp Gly Leu
                   165                 170                 175
   Val Ile Thr Leu Asp Thr Trp Ile Phe Gly Trp Arg Pro Arg Asp Leu
               180                 185                 190
   Thr Ile Ser Asn Phe Pro Phe Leu Arg Gly Leu Cys Leu Thr Asn Tyr
           195                 200                 205
   Val Thr Asp Pro Val Phe Gln Lys Lys Phe Lys Ala His Ser Gly Val
       210                 215                 220
   Glu Ala Glu Gly Leu Arg Asp Asn Pro Arg Leu Ala Ala Asp Phe Trp
   225                 230                 235                 240
   His Gly Leu Phe Gly His Ser Val Thr Trp Glu Asp Ile Asp Trp Val
                   245                 250                 255
   Arg Ser Ile Thr Lys Met Pro Val Ile Leu Lys Gly Ile Gln His Pro
               260                 265                 270
   Asp Asp Ala Arg Arg Ala Val Asp Ser Gly Val Asp Gly Ile Tyr Cys
           275                 280                 285
   Ser Asn His Gly Gly Arg Gln Ala Asn Gly Gly Leu Pro Ala Leu Asp
       290                 295                 300
   Cys Leu Pro Glu Val Val Lys Ala Ser Gly Asp Thr Pro Val Leu Phe
   305                 310                 315                 320
   Asp Ser Gly Ile Arg Thr Gly Ala Asp Val Val Lys Ala Leu Ala Met
                   325                 330                 335
   Gly Ala Ser Ala Val Gly Ile Gly Arg Pro Tyr Ala Trp Gly Ala Ala
               340                 345                 350
   Leu Gly Gly Ser Lys Gly Ile Glu His Val Ala Arg Ser Leu Leu Ala
           355                 360                 365
   Glu Ala Asp Leu Ile Met Ala Val Asp Gly Tyr Arg Asn Leu Lys Glu
       370                 375                 380
   Leu Thr Ile Asp Ala Leu Arg Pro Thr Arg
   385                 390
<210> 2
   <211> 369
   <212> PRT
   <213> Pediococcus acidilactici
<400> 2
   Met Thr Met Ile Asn Gly Tyr Glu Gln Ser Asp Arg Glu Glu Lys Ile
   1               5                   10                  15
   Asp Ile Leu Asn Leu Glu Ser Leu Glu Lys Arg Ala Glu Glu Ile Ile
               20                  25                  30
   Pro Ala Gly Gly Phe Gly Tyr Ile Ala Gly Gly Ser Glu Asp Glu Trp
           35                  40                  45
   Thr Leu Lys Gln Asn Arg Met Ala Phe His His Arg Gln Ile Ala Pro
       50                  55                  60
   Lys Ala Leu Ser Gly Ile Glu Lys Pro Glu Leu Ser Thr Glu Ile Phe
   65                  70                  75                  80
   Gly Ile Pro Leu Asn Thr Pro Val Met Met Ala Pro Ala Ala Ala Gln
                   85                  90                  95
   Gly Leu Ala His Ser Gln Gly Glu Lys Asp Thr Ala Arg Gly Leu Ala
               100                 105                 110
   Ala Val Gly Gly Leu Met Ala Gln Ser Thr Tyr Ser Ser Val Ser Ile
           115                 120                 125
   Ala Glu Thr Ala Ala Ala Gly Gly Asp Ala Ser Gln Phe Phe Gln Leu
       130                 135                 140
   Tyr Met Ser Lys Asp Trp Asn Phe Asn Glu Ser Leu Leu Asp Glu Ala
   145                 150                 155                 160
   Lys Lys Ala Asn Val Lys Ala Ile Ile Leu Thr Val Asp Ala Thr Val
                   165                 170                 175
   Asp Gly Tyr Arg Glu Ala Asp Ile Lys Asn Lys Phe Thr Phe Pro Leu
               180                 185                 190
   Pro Met Ala Asn Leu Ile Lys Phe Ser Glu Gly Asn Gly Gln Gly Lys
           195                 200                 205
   Gly Ile Glu Glu Ile Tyr Ala Ser Ala Ala Gln Asn Ile Arg Pro Glu
       210                 215                 220
   Asp Val Lys Arg Ile Ala Asp Tyr Thr Asn Leu Pro Val Ile Val Lys
   225                 230                 235                 240
   Gly Ile Gln Thr Pro Glu Asp Ala Ile Arg Ala Ile Asp Ala Gly Ala
                   245                 250                 255
   Ala Gly Ile Tyr Val Ser Asn His Gly Gly Arg Gln Leu Asn Gly Gly
               260                 265                 270
   Pro Ala Ser Phe Asp Val Leu Glu Asp Ile Ala Thr Ala Val Asn Lys
           275                 280                 285
   Gln Val Pro Ile Ile Phe Asp Ser Gly Val Arg Arg Gly Ser Asp Val
       290                 295                 300
   Phe Lys Ala Leu Ala Ser Gly Ala Asp Leu Val Ala Leu Gly Arg Pro
   305                 310                 315                 320
   Val Ile Tyr Gly Leu Ala Leu Gly Gly Ala Lys Gly Val Gln Ser Val
                   325                 330                 335
   Phe Glu His Leu Asn His Glu Leu Glu Ile Val Met Gln Leu Ala Gly
               340                 345                 350
   Thr Lys Thr Ile Glu Asp Val Lys Asn Asn Ser Leu Leu Asn Ile Lys
           355                 360                 365
   Tyr
<210> 3
   <211> 320
   <212> PRT
   <213> Lactobacillus plantarum
<400> 3
   Met Ser Ser Met Pro Asn His Gln Lys Val Val Leu Val Gly Asp Gly
   1               5                   10                  15
   Ala Val Gly Ser Ser Tyr Ala Phe Ala Met Ala Gln Gln Gly Ile Ala
               20                  25                  30
   Glu Glu Phe Val Ile Val Asp Val Val Lys Asp Arg Thr Lys Gly Asp
           35                  40                  45
   Ala Leu Asp Leu Glu His Ala Gln Ala Phe Thr Ala Pro Lys Lys Ile
       50                  55                  60
   Tyr Ser Gly Glu Tyr Ser Asp Cys Lys Asp Ala Asp Leu Val Val Ile
   65                  70                  75                  80
   Thr Ala Gly Ala Pro Gln Lys Pro Gly Glu Ser Arg Leu Asp Leu Val
                   85                  90                  95
   Asn Lys Asn Leu Asn Ile Leu Ser Ser Ile Val Lys Pro Val Val Asp
               100                 105                 110
   Ser Gly Phe Asp Gly Ile Phe Leu Val Ala Ala Asn Pro Val Asp Ile
           115                 120                 125
   Leu Thr Tyr Ala Thr Trp Lys Phe Ser Gly Phe Pro Lys Asp Arg Val
       130                 135                 140
   Ile Gly Ser Gly Thr Ser Leu Asp Ser Ser Arg Leu Arg Val Ala Leu
   145                 150                 155                 160
   Gly Lys Gln Phe Asn Val Asp Pro Arg Ser Val Asp Ala Tyr Ile Met
                   165                 170                 175
   Gly Glu His Gly Asp Ser Glu Phe Ala Ala Tyr Ser Thr Ala Thr Ile
               180                 185                 190
   Gly Thr Arg Pro Val Arg Asp Val Ala Lys Glu Gln Gly Val Ser Asp
           195                 200                 205
   Glu Asp Leu Ala Lys Leu Glu Asp Gly Val Arg Asn Lys Ala Tyr Asp
       210                 215                 220
   Ile Ile Asn Leu Lys Gly Ala Thr Phe Tyr Gly Ile Gly Thr Ala Leu
   225                 230                 235                 240
   Met Arg Ile Ser Lys Ala Ile Leu Arg Asp Glu Asn Ala Val Leu Pro
                   245                 250                 255
   Val Gly Ala Tyr Met Asp Gly Gln Tyr Gly Leu Asn Asp Ile Tyr Ile
               260                 265                 270
   Gly Thr Pro Ala Val Ile Gly Gly Thr Gly Leu Lys Gln Ile Ile Glu
           275                 280                 285
   Ser Pro Leu Ser Ala Asp Glu Leu Lys Lys Met Gln Asp Cys Ala Ala
       290                 295                 300
   Thr Leu Lys Lys Val Leu Asn Asp Gly Leu Ala Glu Leu Glu Asn Lys
   305                 310                 315                 320

## Description

The present invention relates to methods for purifying glycoproteins or enveloped viruses comprising glycostructures on their envelopes from a vertebrate cell culture supernatant.

### BACKGROUND OF THE INVENTION

Therapeutic glycoproteins are usually manufactured using a combination of animal cell culture, cell separation, filtration, and chromatographic steps. Integrated steps for viral clearance are also often included. For the majority of therapeutic glycoproteins, the downstream processing still occupies the major part of the overall manufacturing costs. The number of downstream processing steps required to achieve highly purified therapeutic glycoproteins is negatively correlated with overall process yield. Therefore, downstream processes have traditionally been limited to a minimum number of steps. This in turn resulted in a broad adoption of affinity-based capture steps as the first step of modern downstream purification strategies for therapeutic glycoproteins.

The most well-known example is the purification of monoclonal antibody therapeutics by means of protein-A-affinity capture as a first step in downstream processing. Although this method has been widely adopted by industry, it still has some major limitations and drawbacks. High capital expenditure for commercial scale protein-A-columns and immunogenic protein-A-leachables as process-related impurities are just two issues of concern for protein-A-affinity strategies. The most important and still unresolved problem is the elution of bound monoclonal antibodies with low pH elution buffers that can trigger aggregation of the monoclonal antibodies or could cause pH-induced deamidation of critical amino acid side chains or the loss of sialic acid from the glycans by unintentional hydrolytic cleavage of the labile glycosidic bond.

Another downstream process that includes a highly specific capture step is the purification of factor VIII. The classical immunoaffinity capture during factor VIII downstream processing has recently been replaced by a 13 KDa recombinant protein ligand that is eluted by a mix of propylene glycol and arginine. Here, the issue of harsh elution conditions has been partially solved. However, the delicate factor VIII molecule is still exposed to chaotropic arginine that may potentially impact its structural integrity and the chromatographic support functionalized with a recombinant protein is still unfavorably expensive.
Live virions and attenuated live vaccine virions also require a certain level of downstream purification and enrichment. Most enveloped viruses carry a dominant mix of high mannose and some complex-type N-glycans. Thus, they are also amenable to glyco-affinity capture. In order to retain live virions, very mild binding and elution conditions are required.

HENGYE LI et al. (CHEMICAL COMMUNICATIONS, 2011, Vol. 47, No. 28, PAGEs 8169-8171) refer to Wulff-type boronate for boronate affinity capture of cis-diol compounds at medium acidic pH condition. In addition, HENGYE LI et al. (CHEMICAL COMMUNICATIONS, 2012, Vol. 48, No. 34, PAGEs 4115-4117) refer to a benzoboroxole-functionalized monolithic column for the selective enrichment and separation of cis-diol containing biomolecules.

Thus, there is still a need for a method for purifying glycoproteins or enveloped viruses. In particular, a method for purifying glycoproteins or enveloped viruses that allows binding and elution under mild physiological conditions and that is cost-effective would be highly desirable for industrial downstream processing applications.

Boronate is an example for a cheap and effective ligand that has been intensively investigated for its ability to aid in the purification of glycoproteins. Methods for using phenylboronic acid as a selective ligand for purifying glycoproteins have long been described in the literature [1, 2]. These methods allow competitive elution of bound glycoproteins by low molecular weight cis-diol compounds such as sorbitol or other cis-diol sugar alcohols. Although boronate affinity chromatography was introduced in the 1970s, the number of successful applications for purification of glycoproteins at pilot and commercial scale is still limited.

There are three possible explanations for this: (i) Requirement for high-pH binding conditions: Current conventional boronate affinity chromatography media bind glycoproteins only at pH > 8.0. The earliest and still most widely used boronate ligand is 3-aminophenylboronic acid, which has a pKa of 8.8 [3]. In commercial boronate affinity resins containing immobilized m-aminophenylboronate, the meta-amino group is used to couple it to solid supports which further reduces the electron withdrawing effect of the amino group. Therefore, in all applications using immobilized 3-aminophenylboronic acid, the pH should be as high as reasonably possible, usually above 8.5 [3]. Such high pH values for binding and elution of therapeutic glycoproteins are highly undesirable and, thus, render such methods inapplicable for the purification of therapeutic glycoproteins. (ii) Undesired protein-boronate complex formation reduces capacity and purification efficiency of this method: Boronate anions can act as a cation exchanger particularly at a slightly alkaline pH of ∼8. Examples of interactions between boronates and non-glycosylated enzymes such as chymotrypsin or amylase can be found in the literature. One presumption is that the complex formed between the boronate and the enzyme mimics the transition state complex. Other reports emphasize that secondary interactions are responsible for complex formation. Thus, selective elimination of the interactions of the boronate ligand with the protein backbone is necessary to render the method commercially applicable. (iii) Interference of glycoprotein binding to boronate by low molecular weight cis-diols and alpha-hydroxy acids originating from the spent medium: The interaction of boronic acid and its derivatives with cis-diol structures is not only specific for carbohydrates, as any compound containing hydroxyl groups in a proper orientation will form a moderately stable complex with the boronate. As a consequence boronate chromatography can be used for the separation of glycoproteins, nucleosides, catechol compounds, α-hydroxycarboxylic acids as well as aromatic α-hydroxy acids and amides. Cell culture supernatants from industrial vertebrate, e.g. mammalian, producer cell lines contain dominant boronate binding compounds that compete with glycoproteins for the boronate ligand cis-diols and alpha-hydroxy acids. The alpha-hydroxy acid lactate is such a compound that interferes with boronate-affinity [10, 11, 12]. Lactate (2-Hydroxypropionate) can abundantly be present in vertebrate, e.g. mammalian, cell culture supernatants because most vertebrate, e.g. mammalian, producer cell lines still suffer from the Warburg-effect [13] and typically lack the ability to consume lactate to the point where the lactate concentration in the supernatant is zero.

It appears that the first two of these problems have been solved recently. The first problem has been addressed comprehensively by several research groups. New boronate resins including Wulff-Type boronates and Benzoboroxoles have been developed that allow binding and elution of glycoproteins at lower pH [4, 5, 6, 7, 8, 9]. However, surprisingly, these new highly interesting affinity resins have not yet been adopted by industry. This is most likely due to the other two problems mentioned above. The second problem has also been addressed comprehensively by several research groups. It has been found that when Tris(hydroxymethyl)aminomethane (TRIS) is added to the liquid in a boronate chromatography method for the separation of glyco-containing entities, interactions other than glyco-boronate were substantially reduced (see EP 1 280 592). This effect was most surprising in view of the fact that it is recommended in the literature to avoid using TRIS and other compounds containing polyhydroxyls during boronate chromatography, since such substances can reduce the binding capacity of the boronate matrix by direct competition.

The third problem, however, still remained unsolved and has prevented the use of the desirable glyco-affinity for glycoprotein capture at commercial scale. Industrial scale affinity chromatography utilizing the glycomoiety of therapeutic glycoproteins is extremely desirable as it offers high selectivity and allows an economically feasible competitive elution at physiological pH. Thereby, this type of affinity chromatography supports a high degree of first step purification while simultaneously maintaining product integrity during downstream processing. In addition, such synthetic chemical ligands are far cheaper than recombinant protein ligands by several orders of magnitude. Thus, there is a strong need for a cost-efficient process allowing direct affinity capture of these molecules from cell culture supernatant via affinity-capture and gentle but affordable competitive elution at near neutral pH.

The present invention discloses such a method. The inventor of the present invention provides a method for purifying glycoproteins, wherein a vertebrate cell culture supernatant which is free of lactate or has a reduced amount of lactate is applied on a solid support in the glycoprotein capture step. In particular, the inventor of the present invention found that the addition of a lactate converting enzyme to the vertebrate cell culture supernatant directly preceding the glycoprotein capture step using a solid support reduces the amount of lactate, in the best case to zero, and, thus, allows the direct capture of the glycoproteins from cell culture supernatant and their cost efficient separation in high purity. In this way, the interference of the binding of glycoproteins to a ligand having affinity for a pyranoid-diol functionality, particularly 1,2 cis-diol or 1,3 trans-diol functionality, and/or alpha-hydroxy acid functionality, in the glycoprotein capture step by lactate comprised in the vertebrate cell culture supernatant and originating from the spent medium can be removed.

### SUMMARY OF THE INVENTION

In a first aspect, the present invention relates to a method for purifying glycoproteins or enveloped viruses comprising glycostructures on their envelopes from a vertebrate cell culture supernatant comprising the steps of:
(i) providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) applying the vertebrate cell culture supernatant to a solid support comprising a chemical compound capable of binding to the glycoproteins or glycostructures on the envelopes of the enveloped viruses,
wherein the chemical compound capable of binding to the glycoproteins or glycostructures on the envelopes of the enveloped viruses is
a boronate, or
a complex of a metal and a glycoside, and
wherein step (ii) is conducted at a pH between 6 and 8.

In a second aspect, the present invention relates to a method for purifying glycoproteins or enveloped viruses comprising glycostructures on their envelopes from a vertebrate cell culture supernatant comprising the steps of:
(i) providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) precipitating the glycoproteins or enveloped viruses comprising glycostructures on their envelopes from the vertebrate cell culture supernatant with a chemical compound capable of binding to said glycoproteins or glycostructures,
wherein the chemical compound capable of binding to the glycoproteins or glycostructures on the envelopes of the enveloped viruses is
a boronate, or
a complex of a metal and a glycoside, and
wherein step (ii) is conducted at a pH between 6 and 8.

Further described herein but not encompassed by the present invention is a vertebrate cell for producing a glycoprotein or an enveloped virus comprising glycostructures on its envelope, said cell comprises
a polynucleotide sequence comprising a first nucleotide sequence encoding a lactate converting enzyme and a second nucleotide sequence encoding a signal peptide, wherein the signal peptide is capable of directing the lactate converting enzyme to the culture medium,
wherein said cell expresses said enzyme and said signal peptide.

Further described herein but not encompassed by the present invention is a method for purifying glycoproteins or enveloped viruses comprising glycostructures on their envelopes comprising the steps of:
(i) cultivating the vertebrate cell of the third aspect in a culture medium, and
(ii) separating the vertebrate cell from the culture medium and thereby obtaining a lactate-free vertebrate cell culture supernatant comprising glycoproteins or enveloped viruses comprising glycostructures on their envelopes.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

The term "comprise" or variations such as "comprises" or "comprising" according to the present invention means the inclusion of a stated integer or group of integers but not the exclusion of any other integer or group of integers. The term "consisting essentially of" according to the present invention means the inclusion of a stated integer or group of integers, while excluding modifications or other integers which would materially affect or alter the stated integer. The term "consisting of" or variations such as "consists of" according to the present invention means the inclusion of a stated integer or group of integers and the exclusion of any other integer or group of integers.

The terms "polypeptide" and "protein" are used interchangeably in the context of the present invention and refer to a long peptide-linked chain of amino acids, e.g. one that is typically 50 amino acids long or longer than 50 amino acids.

In the context of the present invention, the term "oligopeptide" refers to a short peptide-linked chain of amino acids, e.g. one that is typically less than about 50 amino acids long and more typically less than about 30 amino acids long. Said short peptide-linked chain of amino acids is typically more than about 2 amino acids long.

In the context of the present invention, the term "fusion protein" refers to a polypeptide comprising a polypeptide or polypeptide fragment coupled to heterologous amino acid sequences. Fusion proteins are useful because they can be constructed to contain two or more desired functional elements from two or more different proteins.

The terms "antibody", "immunoglobulin", "Ig" and "Ig molecule" are used interchangeably in the context of the present invention. The CH2 domain of each heavy chain contains a single site for N-linked glycosylation at an asparagine residue linking an N-glycan to the antibody molecule, usually at residue Asn-297 (Kabat et al., Sequence of proteins of immunological interest, Fifth Ed., U.S. Department of Health and Human Services, NIH Publication No. 91-3242). Included within the scope of the term are classes of Igs, namely, IgG, IgA, IgE, IgM, and IgD. Also included within the scope of the terms are the subtypes of IgGs, namely, IgG1, IgG2, IgG3 and IgG4. The terms are used in their broadest sense and include monoclonal antibodies (including full length monoclonal antibodies), polyclonal antibodies, single chain antibodies, and multispecific antibodies (e.g. bispecific antibodies).

The term "antibody fragment", as used herein, refers to a fragment of an antibody that contains at least the portion of the CH2 domain of the heavy chain immunoglobulin constant region which comprises an N-linked glycosylation site of the CH2 domain and is capable of specific binding to an antigen, i.e. chains of at least one V_{L} and/or V_{H}-chain or binding part thereof.

The terms "Fc domain" and "Fc region", as used herein, refer to a C-terminal portion of an antibody heavy chain that interacts with cell surface receptors called Fc receptors and some proteins of the complement system. This property allows antibodies to activate the immune system.

In the context of the present invention, the term "glycoprotein" refers to proteins that contain oligosaccharide chains (glycans) covalently attached to their polypeptide side-chains. The carbohydrate is attached to the protein in a co-translational or posttranslational modification. This process is known as glycosylation such as N-glycosylation or O-glycosylation.

In the context of the present invention, the term "glycostructure" encompasses glycoproteins and/or glycooligopeptides that contain oligosaccharide chains (glycans) covalently attached to their polypeptide/oligopeptide side-chains. The carbohydrate is attached to the structure in a co-translational or posttranslational modification. As mentioned above, this process is known as glycosylation such as N-glycosylation or O-glycosylation.

The term "N-glycosylation", as used herein, means the addition of sugar chains which to the amide nitrogen on the side chain of asparagine. The term "O-glycosylation", as used herein, means the addition of sugar chains on the hydroxyl oxygen on the side chain of hydroxylysine, hydroxyproline, serine, or threonine.

The term "N-glycan", as used herein, means an N-linked polysaccharide or oligosaccharide. An N-linked oligosaccharide is for example one that is or was attached by an N-acetylglucosamine residue linked to the amide nitrogen of an asparagine residue in a protein. The predominant sugars found on N-glycoproteins are glucose, galactose, mannose, fucose, N-acetylgalactosamine (GalNAc), N-acetylglucosamine (GlcNAc) and sialic acid (e.g., N-acetylneuraminic acid (NANA)). The processing of the sugar groups occurs co-translationally in the lumen of the ER and continues in the Golgi apparatus for N-linked glycoproteins. N-glycans have a common pentasaccharide core of Man₃GlcNAc₂ ("Man" refers to mannose; "Glc" refers to glucose; and "NAc" refers to N-acetyl; GlcNAc refers to N-acetylglucosamine). N-glycans differ with respect to the number of branches (antennae) comprising peripheral sugars (e.g., GlcNAc, galactose, fucose and sialic acid) that are added to the Man₃GIcNAc₂ core structure which is also referred to as the "trimannose core", the "pentasaccharide core" or the "paucimannose core". N-glycans are classified according to their branched constituents (e.g., high mannose, complex or hybrid).

The term "O-glycan", as used herein, means an O-linked polysaccharide or oligosaccharide. O-linked glycans are usually attached to the peptide chain through serine or threonine residues. O-linked glycosylation is a true post-translational event which occurs in the Golgi apparatus and which does not require a consensus sequence and no oligosaccharide precursor is required for protein transfer. The most common type of O-linked glycans contain an initial GalNAc residue (or Tn epitope), these are commonly referred to as mucin-type glycans. Other O-linked glycans include glucosamine, xylose, galactose, fucose, or mannose as the initial sugar bound to the Ser/Thr residues. O-Linked glycoproteins are usually large proteins (> 200 kDa) that are commonly biantennary with comparatively less branching than N-glycans.

The term "enveloped viruses comprising glycostructures on their envelopes", as used herein, refers to viruses having viral envelopes covering their protective protein capsids. The envelopes typically are derived from portions of the host cell membranes (phospholipids and proteins), but include some (viral) glycostructures such as glycoproteins and/or glycooligopeptides. Functionally, viral envelopes help viruses to enter host cells and may help them to avoid the host immune system. (Viral) glycostructures such as glycoproteins and/or glycooligopeptides on the surface of the envelopes serve to identify and bind to receptor sites on the host's membrane. The viral envelope then fuses with the host's membrane, allowing the capsid and viral genome to enter and infect the host. The influenza virus and many animal viruses are enveloped viruses.

As mentioned above, lactate (2-hydroxypropionate) can abundantly be present in vertebrate cell culture supernatants because most of the industrial vertebrate producer cell lines presently used still suffer from the Warburg-effect and typically lack the ability to consume lactate to the point where the lactate concentration in the vertebrate cell culture supernatant is remarkably reduced, preferably is zero. The lactate contained in the vertebrate cell culture supernatants from industrial vertebrate producer cell lines such as mammalian producer cell lines competes with the glycoproteins for the ligand, e.g. boronate, comprised on the solid support in the glycoprotein capture step. Particularly, said lactate competes with the glycoproteins for the cis-diol, trans-diol, and/or alpha-hydroxy acid functionalities of said ligand, e.g. boronate, comprised on the solid support.

The inventor of the present invention provides a method for purifying glycoproteins, wherein a vertebrate cell culture supernatant which is free of lactate or has a reduced amount of lactate is applied on a solid support in the glycoprotein capture step. In particular, the inventor of the present invention found that the addition of a lactate converting enzyme to the vertebrate cell culture supernatant directly preceding the glycoprotein capture step using a solid support reduces the amount of lactate, in the best case to zero, and, thus, allows the separation of the glycoproteins in high purity and very cost efficient. In this way, the interference of the binding of glycoproteins to a ligand having affinity for a pyranoid-diol functionality, particularly a 1,2 cis-diol or 1,3 trans-diol functionality, and/or alpha-hydroxy acid functionality in the glycoprotein capture step by lactate comprised in the vertebrate cell culture supernatant and originating from the spent medium can be removed.

Thus, in a first aspect, the present invention relates to a method for purifying glycoproteins or enveloped viruses comprising glycostructures on their envelopes from a vertebrate cell culture supernatant comprising the steps of:
(i) providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) applying the vertebrate cell culture supernatant to a solid support comprising a chemical compound capable of binding to the glycoproteins or glycostructures on the envelopes of the enveloped viruses,
wherein the chemical compound capable of binding to the glycoproteins or glycostructures on the envelopes of the enveloped viruses is
a boronate, or
a complex of a metal and a glycoside, and
wherein step (ii) is conducted at a pH between 6 and 8.

In a second aspect, the present invention relates to a method for purifying glycoproteins or enveloped viruses comprising glycostructures on their envelopes from a vertebrate cell culture supernatant comprising the steps of:
(i) providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) precipitating the glycoproteins or enveloped viruses comprising glycostructures on their envelopes from the vertebrate cell culture supernatant with a chemical compound capable of binding to said glycoproteins or glycostructures,
wherein the chemical compound capable of binding to the glycoproteins or glycostructures on the envelopes of the enveloped viruses is
a boronate, or
a complex of a metal and a glycoside, and
wherein step (ii) is conducted at a pH between 6 and 8.

The methods of both aspects only differ in process step (ii) from each other. Thus, if not otherwise indicated, the following preferred embodiments belong to the methods of the first as well as second aspect of the present invention.

Preferably, the vertebrate cell culture supernatant provided in step (i) of the method of the first or second aspect of the present invention is generated by contacting a vertebrate cell culture supernatant comprising glycoproteins or enveloped viruses comprising glycostructures on their envelopes and lactate with a lactate converting enzyme. Said contacting may be achieved by adding the lactate converting enzyme to the vertebrate cell culture supernatant.

Thus, in a preferred embodiment of the first aspect of the present invention, the method for purifying glycoproteins or enveloped viruses comprising glycostructures on their envelopes from a vertebrate cell culture supernatant comprises the steps of:
(i) contacting a vertebrate cell culture supernatant comprising glycoproteins or enveloped viruses comprising glycostructures on their envelopes and lactate with a lactate converting enzyme and thereby providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) applying the vertebrate cell culture supernatant to a solid support comprising a chemical compound capable of binding to the glycoproteins or glycostructures on the envelopes of the enveloped viruses,
wherein the chemical compound capable of binding to the glycoproteins or glycostructures on the envelopes of the enveloped viruses is
a boronate, or
a complex of a metal and a glycoside, and
wherein step (ii) is conducted at a pH between 6 and 8.

In a preferred embodiment of the second aspect of the present invention, the method for purifying glycoproteins or enveloped viruses comprising glycostructures on their envelopes from a vertebrate cell culture supernatant comprises the steps of:
(i) contacting a vertebrate cell culture supernatant comprising glycoproteins or enveloped viruses comprising glycostructures on their envelopes and lactate with a lactate converting enzyme and thereby providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) precipitating the glycoproteins or enveloped viruses comprising glycostructures on their envelopes from the vertebrate cell culture supernatant with a chemical compound capable of binding to said glycoproteins or glycostructures,
wherein the chemical compound capable of binding to the glycoproteins or glycostructures on the envelopes of the enveloped viruses is
a boronate, or
a complex of a metal and a glycoside, and
wherein step (ii) is conducted at a pH between 6 and 8.

The vertebrate cell culture supernatant comprising glycoproteins or enveloped viruses comprising glycostructures on their envelopes and lactate is obtained from a vertebrate cell culture. Techniques to harvest the vertebrate cell culture supernatant from a vertebrate cell culture are known to the skilled person. This can be done, for example, by centrifugation, sedimentation and/or filtration techniques.

The term "vertebrate cell culture supernatant comprising lactate", as used herein, means a vertebrate cell culture supernatant which usually encompasses lactate. As mentioned above, lactate can abundantly be present in vertebrate cell culture supernatants because most of the industrial vertebrate producer cell lines presently used suffer from the Warburg-effect and typically lack the ability to consume lactate to the point where the lactate concentration in the vertebrate cell culture supernatant is remarkably reduced, preferably is zero. In the context of the present invention, the term "vertebrate cell culture supernatant comprising lactate" refers to a non-treated vertebrate cell culture supernatant. It still comprises lactate. This means that said vertebrate cell culture supernatant has not yet been treated in a way that lactate is reduced, e.g. to zero.

The term "vertebrate cell culture supernatant which is free of lactate", as used herein, means a vertebrate cell culture supernatant which usually comprises lactate, but which does not comprise lactate anymore. Expressed in terms of purity, "a vertebrate cell culture supernatant which is free of lactate", as used herein, means that said supernatant is to 100% free of lactate. In the context of the present invention, the term "vertebrate cell culture supernatant which is free of lactate" refers to a treated vertebrate cell culture supernatant. Such a vertebrate cell culture supernatant is preferably achieved by harvesting a vertebrate cell culture supernatant comprising lactate from a vertebrate cell culture and treating the supernatant by adding a lactate converting enzyme to said supernatant.

The term "vertebrate cell culture supernatant which is substantially free of lactate", as used herein, means a vertebrate cell culture supernatant which usually comprises lactate, but which is to at least about 95%, about 96%, about 97%, about 98%, about 99%, about 99.1%, about 99.2%, about 99.3%, about 99.4%, about 99.5%, about 99.6%, about 99.7%, about 99.8%, or at least about 99.9% free of lactate. As mentioned above, such a vertebrate cell culture supernatant is preferably achieved by harvesting a vertebrate cell culture supernatant comprising lactate from a vertebrate cell culture and treating the supernatant by adding a lactate converting enzyme to said supernatant.

The term "vertebrate cell culture supernatant which has a reduced amount of lactate as compared to a reference vertebrate cell culture supernatant" is used herein to indicate that a vertebrate cell culture supernatant which usually comprises lactate has a reduced amount of lactate when compared to a reference vertebrate cell culture supernatant. The term "vertebrate cell culture supernatant which has a reduced amount of lactate" refers to a treated vertebrate cell culture supernatant. The term "treatment", as used herein, means a procedure that reduces, e.g. to zero, the amount of lactate in a vertebrate cell culture supernatant. A vertebrate cell culture supernatant which has a reduced amount of lactate is preferably achieved by harvesting a vertebrate cell culture supernatant comprising lactate from a vertebrate cell culture and treating the supernatant by adding a lactate converting enzyme to said supernatant. The "reference vertebrate cell culture supernatant" is the same vertebrate cell culture supernatant harvested from the vertebrate cell culture, but is non-treated. Regarding the use of a lactate converting enzyme, this means that the "reference vertebrate cell culture supernatant" is the same vertebrate cell culture supernatant harvested from the vertebrate cell culture, but is non-treated with a lactate converting enzyme.

It is preferred that lactate is reduced in the vertebrate cell culture supernatant between about 10% to 99,9%, preferably about 15%, about 20%, about 25%, about 30%, about 35%, 40%, about 45%, about 50%, about 55%, about 60%, about 65%, about 70%, about 75%, about 80%, about 85%, about 86%, about 87%, about 88%, about 89%, about 90%, about 91%, about 92%, about 93%, about 94%, about 95%, about 96%, about 97%, about 98%, about 99%, about 99.5%, or about 99.9%, compared to the reference vertebrate cell culture supernatant. Usual end-of-production (EOP) lactate concentrations for glycoprotein purification processes amount to about 2.0 g/l, e.g. 2.0 g/l. Thus, it is preferred that the lactate concentration is reduced in the vertebrate cell culture supernatant below 1.5 g/l, more preferably below 1.0 g/l, even more preferably below 0.5 g/l, most preferably below 0.1 g/l, e.g. below 2.0 g/l, below 1.9 g/l, below 1.8 g/l, below 1.7 g/l, below 1.6 g/l, below 1.5 g/l, below 1.4 g/l, below 1.3 g/l, below 1.2 g/l, below 1.1 g/l, below 1.0 g/l, below 0.9 g/l, below 0.8 g/l, below 0.7 g/l, below 0.6 g/l, below 0.5 g/l, below 0.4 g/l, below 0.3 g/l, below 0.2 g/l, or below 0.1 g/l.

The skilled person can easily determine experimentally the amount of lactate in a vertebrate cell culture supernatant. This can be done, for example, with enzymatic amperometric sensors used for example in the BioPAT® Trace process (Sartorius Stedim Biotech) or with the YSI 2950 biochemical analyzer (Kreienbaum Wissenschaftliche Meßsysteme e.K).

It is preferred that the glycoproteins purified with the method of the first or second aspect of the present invention are glycoproteins comprising a pyranoid-diol functionality, preferably a 1,2 cis-diol and/or 1,3 trans-diol functionality. It is, alternatively or additionally, preferred that the glycoproteins are proteins of the N-glycan type. The glycoproteins may further be antibodies, antibody fragments, fusion proteins, virus proteins, virus protein fragments, antigens, cytokines, clotting factors, growth factors, enzymes, or hormones. Preferably, the antibody or the antibody fragment is selected from the group consisting of IgG, preferably IgG1, IgG2, IgG3, IgG4, IgM, IgA, IgD and IgE. Preferably, the fusion protein comprises the Fc region of an antibody, e.g. the Fc region of IgG, such as the Fc region of IgG1, IgG2, IgG3, IgG4, IgM, IgA, IgD or IgE. Preferably, the cytokine is interferon or interleukin. Preferably, the clotting factor is factor VII, factor IX, or factor VIII, in particular full-length and B-domain deleted versions thereof. Preferably, the enzyme is a glucocerebrosidase, an iduronidase, arylsulfatase A and B, alpha-glucosidase, or iduronat-2-sulfatase. Preferably, the hormone is a follicle stimulating hormone (FSH), luteinizing hormone (LH), chorionic gonadotropin (HCG), or a tyroid stimulating hormone (TSH). It is particularly preferred that the virus proteins or virus protein fragments are comprised in the envelope membrane of an enveloped virus. Said enveloped virus may be an influenza virus, a rabies virus, chikungunya virus, vaccinia virus including modified vaccinia ankara virus, mumps virus, measles virus, canine distemper virus, or a peste des petits ruminants virus (PPRV).

As mentioned above, the enveloped viruses purified with the method of the first or second aspect of the present invention comprise glycostructures, e.g. glycoproteins, on their envelopes. Said glycostructures, e.g. glycoproteins, are preferably glycostructures, e.g. glycoproteins, comprising a pyranoid-diol functionality, preferably a 1,2 cis-diol and/or 1,3 trans-diol functionality. It is, alternatively or additionally, preferred that the glycostructures, e.g. glycoproteins, are glycostructures, e.g. glycoproteins, of the N-glycan type. The glycoproteins may be virus proteins or virus protein fragments. Preferably, the enveloped virus comprising glycostructures on its envelope is an influenza virus, a rabies virus, chikungunya virus, vaccinia virus including modified vaccinia ankara virus, mumps virus, measles virus, canine distemper virus, or a peste des petits ruminants virus (PPRV).

In a preferred embodiment, the method of the first aspect of the present invention further comprises the step(s) of:
(iii) washing the solid support with a washing buffer, and/or
(iv) releasing the glycoproteins or enveloped viruses comprising glycostructures on their envelopes from the solid support with an elution buffer containing a competitive eluent.

The solid support may be a membrane or a solid phase chromatographic support such as a resin or a monolith. The solid support may be in the format of a chromatography column or a membrane adsorber. The solid support may also be a bead, particularly a set of beads.

The washing buffer may be any buffer which allows the removal of unspecific binding partners from the solid support. As washing buffer, standard phosphate buffered saline (PBS) may be used.

The elution buffer may be any buffer which allows the release of the glycoproteins or enveloped viruses comprising glycostructures on their envelopes from the solid support. As elution buffer, standard PBS supplemented with fructose may be used.

The competitive eluent used in step (iv) of the method of the first aspect of the present invention is any eluent which allows the displacement of the glycoproteins or enveloped viruses comprising glycostructures on their envelopes from the solid support. It is preferred that the competitive eluent comprises a furanoid-diol functionality, preferably a 1,2 cis-diol or 1,3 trans-diol functionality, a pyranoid-diol functionality, preferably a 1,2 cis-diol or 1,3 trans-diol functionality, and/or an alpha-hydroxy acid functionality. Preferably, the competitive eluent is selected from the group consisting of furanoid cis diol sugars or glycosides, pyranoid 1,2,3 triol sugars or glycosides, sugar alcohols, preferably sorbitol, adonitol, mannitol, xylitol, dulcitol, pentaerythritol, or erythritol, alpha hydroxy carbonic acids, preferably glycolate, lactate, malate, tartrate, citrate, alpha-hydroxy caprylate, salicylate or glucuronate, and hydroxylamino compounds, preferably triethanolamin or Tris-hydroxymethyl-aminomethan (TRIS).

In a preferred embodiment, the method of the second aspect of the present invention further comprises the step(s) of:
(iii) washing the precipitate with a washing buffer, and/or
(iv) releasing the glycoproteins or enveloped viruses comprising glycostructures on their envelopes from the precipitate with an elution buffer (dissociating buffer) containing a competitive eluent (competitive dissociating reactant).

The washing buffer may be any buffer which allows the removal of unspecific binding partners from the precipitate. As washing buffer, phosphate buffered saline (PBS) may be used.

The elution buffer may be any buffer which allows the dissociation of the precipitate and, thus, the release of the glycoproteins or enveloped viruses comprising glycostructures on their envelopes from the precipitate. Thus, it may also be designated as dissociation buffer. As elution buffer (dissociation buffer), phosphate buffered saline supplemented with fructose may be used.

The competitive eluent used in step (iv) of the method of the second aspect of the present invention is any eluent which allows the displacement of the glycoproteins or enveloped viruses comprising glycostructures on their envelopes from the precipitate. It is further capable of dissociating the precipitate. Thus, it may also be designated as competitive dissociating reactant. It is preferred that the competitive eluent (competitive dissociating reactant) comprises a furanoid-diol functionality, preferably a 1,2 cis-diol or 1,3 trans-diol functionality, a pyranoid-diol functionality, preferably a 1,2 cis-diol or 1,3 trans-diol functionality, and/or an alpha-hydroxy acid functionality. Preferably, the competitive eluent (competitive dissociating reactant) is selected from the group consisting of furanoid cis diol sugars or glycosides, pyranoid 1,2,3 triol sugars or glycosides, sugar alcohols, preferably sorbitol, adonitol, mannitol, xylitol, dulcitol, pentaerythritol, or erythritol, alpha hydroxy carbonic acids, preferably glycolate, lactate, malate, tartrate, citrate, alpha-hydroxy caprylate, salicylate or glucuronate, and hydroxylamino compounds, preferably triethanolamin or Tris-hydroxymethyl-aminomethan (TRIS).

Preferably, steps (iii), and/or (iv) of the method of the first or second aspect of the present invention are conducted at a pH between 6 and 8, more preferably at a pH between 6.5 and 7.5, even more preferably at a pH between 6.8 and 7.2, e.g. at a neutral pH (pH 7). This means that it is preferred that the washing and/or elution (dissociation) buffer have such a pH. This also means that the binding of the glycoproteins or enveloped viruses comprising glycostructures on their envelopes to the solid support is performed at such a pH or that the precipitation of the glycoproteins or enveloped viruses comprising glycostructures on their envelopes from the vertebrate cell culture supernatant is performed at such a pH.

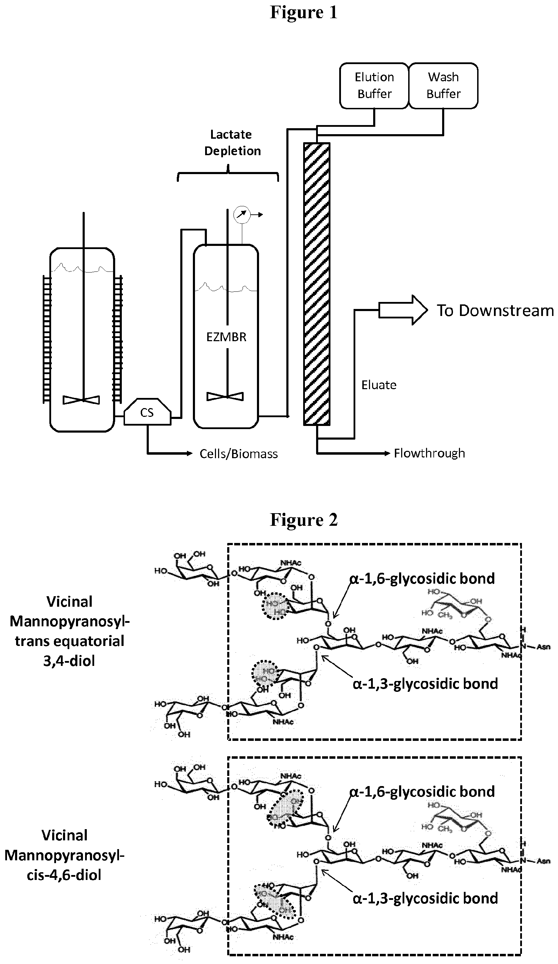

The method of the first or second aspect of the present invention is preferably carried out in an enzyme bioreactor (EZBR) assembly as shown in **Figure 1****.**

The skilled person is aware of techniques how to determine the purity of the glycoproteins or enveloped viruses purified with the method of the first or second aspect of the present invention. The purity of the glycoproteins is preferably measured using (i) spectrometry, preferably mass spectrometry (MS), (ii) chromatography, preferably liquid chromatography (LC), more preferably high performance liquid chromatography (HPLC), (iii) gel electrophoresis, preferably SDS gel electrophoresis, (iv) Westernblot/Immunoblot, or (v) combinations thereof. It is preferred that the chromatography, preferably liquid chromatography (LC), more preferably high performance liquid chromatography (HPLC), is combined with spectrometry, preferably mass spectrometry (MS). Accordingly, the purity of the purified glycoprotein is preferably measured using liquid chromatography-mass spectrometry (LC-MS) and is more preferably measured using high performance liquid chromatography-mass spectrometry (HPLC-MS).

Vertebrate cell culture supernatants from industrial vertebrate producer cell lines contain dominant lactate that competes with the glycoproteins for the binding positions of the ligand in the glycoprotein capture step. Said ligand has an affinity for the pyranoid-diol functionality, particularly 1,2 cis-diol and/or 1,3 trans-diol functionality, of the glycoproteins as well as an affinity for the alpha-hydroxy acid functionality of the lactate. Lactate is the most dominant interfering substance in vertebrate cell culture supernatants that renders direct capture of the glycoproteins impractical. The removal of lactate from the vertebrate cell culture supernatant is, therefore, an immediate prerequisite for enabling direct capture of glycoproteins. As mentioned above, lactate accumulates in the vertebrate cell culture as most industrial vertebrate producer cell lines still suffer from the Warburg-effect and typically lack the ability to consume lactate.

The term "lactate converting enzyme", as used herein, refers to any enzyme which is capable of degrading or depleting lactate. Lactate can also be designated as 2-hydroxy-propanoate. In particular, the term "lactate converting enzyme", as used herein, refers to any enzyme which is capable of converting lactate to a metabolic product which does not compete anymore with the glycoproteins or with the glycostructures on the envelopes of the enveloped viruses for the binding positions of the chemical compound mentioned in step (ii) of the method of the first or second aspect of the present invention. The term "lactate converting enzyme", as used herein, further refers to any enzyme which is capable of converting lactate to a metabolic product that does not comprise an alpha-hydroxy acid functionality anymore. In the context of the present invention, said chemical compound is a compound which is capable of binding to the glycoproteins, particularly to the glycostructures of the glycoproteins, or is capable of binding to the glycostructures on the envelopes of the enveloped viruses. In particular, said chemical compound is capable of binding to the pyranoid-diol functionality of said glycoproteins or glycostructures, preferably to the 1,2 cis-diol and/or 1,3 trans-diol functionality of said glycoproteins or glycostructures. In the context of the present invention, said chemical compound is also capable of binding to lactate. In particular, said chemical compound is capable of binding to the alpha-hydroxy acid functionality of said lactate. Thus, said chemical compound is capable of binding to pyranoid-diol functionalities, preferably 1,2 cis-diol and/or 1,3 trans-diol functionalities, as well as alpha-hydroxy acid functionalities. More specifically, the glycoproteins or glycostructures on the envelopes of the enveloped viruses comprise pyranoid-diol functionalities, preferably 1,2 cis-diol and/or 1,3 trans-diol functionalities, which allow the binding to said chemical compound, e.g. comprised on the solid support, and lactate comprises an alpha-hydroxy acid functionality which allows the binding to said chemical compound, e.g. comprised on the solid support. The metabolic products of lactate, e.g. acetate, carbon dioxide, and water produced by the lactate-2-monooxygenase, or pyruvate and hydrogen peroxide produced by the lactate-oxidase, however, do not comprise said functionality anymore. They are, thus, not able anymore to bind to said chemical compound, e.g. comprised on the solid support. In view of its properties to degrade or deplete lactate in the vertebrate cell culture supernatant, the "lactate converting enzyme" can also be designated as "lactate degrading enzyme" or "lactate depleting enzyme".

In a preferred embodiment of the method of the first or second aspect of the present invention, the lactate converting enzyme is a (S)-2-hydroxy-acid oxidase or a lactate dehydrogenase.

The lactate dehydrogenase is an enzyme that catalyzes the conversion of lactate into pyruvate with or without concomitant conversion of NAD⁺ into NADH. It is preferred that the lactate dehydrogenase is from Lactobacillus plantarum.

The (S)-2-hydroxy-acid oxidase as lactate converting enzyme is preferably a decarboxylating lactate oxidizing enzyme. It decarboxylates lactate into acetate, carbon dioxide, and water.

In a more preferred embodiment of the method of the first or second aspect of the present invention, the (S)-2-hydroxy-acid oxidase is a lactate-2-monooxygenase or a lactate oxidase, most preferably a lactate-2-monooxygenase, e.g. a decarboxylating lactate-2-monooxygenase.

The decarboxylating lactate-2-monooxygenase is an enzyme that converts the lactate (2-hydroxy-propanoate) into acetate, carbon dioxide, and water. Said metabolic products do not comprise an alpha-hydroxy acid functionality anymore. It is preferred that the lactate-2-monooxygenase is from Mycobacterium smegmatis. In order to ensure complete lactate conversion, either the lactate concentration can be monitored directly or the carbon dioxide concentration in the enzyme reactor can be monitored during this enzyme reaction until a peak of carbon dioxide concentration is reached.

The lactate-oxidase is an enzyme that converts the lactate (2-hydroxy-propanoate) into pyruvate and hydrogen peroxide. Said metabolic products do not comprise an alpha-hydroxy acid functionality anymore. However, in different purification processes of glycoproteins or enveloped viruses, hydrogen peroxide is an unwanted or non-tolerable metabolic product. In these cases, the lactate oxidase is preferably used in combination with a peroxidase. The peroxidase converts hydrogen peroxide into water. Said peroxidase is preferably selected from the group consisting of L-ascorbate peroxidase, guiacol peroxidase (2-methoxyphenol peroxidase), glutathione peroxidase, and peroxiredoxin. If the L-ascorbate peroxidase is used in combination with the lactate oxidase in the method of the first or second aspect of the present invention, L-ascorbate is additionally added to the vertebrate cell supernatant. If the guiacol peroxidase (2-methoxyphenol peroxidase) is used in combination with the lactate oxidase in the method of the first or second aspect of the present invention, 2-methoxyphenol is additionally added to the vertebrate cell supernatant. It is further preferred that the lactate oxidase is from Pediococcus sp.

The lactate-2-monooxygenase has preferably the sequence according to SEQ ID NO: 1 or is a variant thereof. It is a decarboxylating lactate-2-monooxygenase. The lactate-oxidase has preferably the sequence according to SEQ ID NO: 2 or is a variant thereof. The lactate dehydrogenase has preferably the sequence according to SEQ ID NO: 3 or is a variant thereof.

A lactate-2-monooxygenase, lactate-oxidase, or lactate dehydrogenase variant which is preferred in the present invention differs from the lactate-2-monooxygenase, lactate-oxidase, or lactate dehydrogenase from which it is derived by up to 150 (i.e. up to 1, 2, 3, 4, 5, 6, 7, 8, 9, 10, 11, 12, 13, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, or 150) amino acid changes in the amino acid sequence (i.e. exchanges, insertions, deletions, N-terminal truncations and/or C-terminal truncations). The amino acid exchanges may be conservative or non-conservative. A lactate-2-monooxygenase, lactate-oxidase, or lactate dehydrogenase variant which is preferred in the present invention can alternatively or additionally be characterised by a certain degree of sequence identity to the lactate-2-monooxygenase, lactate-oxidase, or lactate dehydrogenase from which it is derived. Thus, the lactate-2-monooxygenase, lactate-oxidase, or lactate dehydrogenase variant which is preferred in the present invention has a sequence identity of at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to the respective reference lactate-2-monooxygenase, lactate-oxidase, or lactate dehydrogenase. Preferably, the sequence identity is over the whole length of the respective reference lactate-2-monooxygenase, lactate-oxidase, or lactate dehydrogenase.

Additionally, a lactate-2-monooxygenase, lactate-oxidase, or lactate dehydrogenase variant having the above indicated degree of relatedness to the reference enzyme is only regarded as a variant in the context of the present invention, if it exhibits its relevant biological activity to a degree of at least 30%, preferably of at least 50%, more preferably of at least 80%, of the biological activity of the respective reference enzyme. The relevant "biological activity" in the context of the present invention is the "enzyme activity", i.e. the activity of the lactate-2-monooxygenase variant to utilize the substrate lactate and coverts it into acetate, carbon dioxide, and water, the activity of the lactate-oxidase variant to utilize the substrate lactate and converts it into pyruvate and hydrogen peroxide, or the activity of the lactate dehydrogenase to utilize the substrate lactate and converts it into pyruvate. The skilled person can readily assess whether a lactate-2-monooxygenase, lactate-oxidase, or lactate dehydrogenase variant has an enzyme activity of at least 30%, preferably of at least 50%, more preferably of at least 80%, of the enzyme activity of the respective reference lactate-2-monooxygenase, lactate-oxidase, or lactate dehydrogenase. Suitable assays, e.g. enzyme activity assays, for determining the "enzyme activity" of the enzyme variant compared to the "enzyme activity" of the respective reference enzyme are known to the skilled person.

After completion of lactate conversion, the lactate depleted vertebrate cell supernatant is directly submitted to glycoprotein or glycostructure capture where the glycoproteins or glycostructures are separated from impurities via a solid support, e.g. solid column such as affinity chromatography column, utilizing the pyranoid-diol functionality of said glycoproteins or glycostructures, more preferably the 1,2 cis-diol and/or 1,3 trans-diol functionality of said glycoproteins or glycostructures. Preferably, said glycoproteins or glycostructures are glycoproteins or glycostructures of the N-glycan type.

As mentioned above, the chemical compound mentioned in step (ii) of the method of the first or second aspect of the present invention is capable of binding to the/said glycoproteins or glycostructures on the envelopes of the enveloped viruses. It particularly binds to the glycostructures of the/said glycoproteins. In a preferred embodiment of the method of the first or second aspect of the present invention, the chemical compound is capable of binding to the pyranoid-diol functionality of the/said glycoproteins or glycostructures. In a more preferred embodiment of the method of the first or second aspect of the present invention, the chemical compound is capable of binding to the 1,2 cis-diol and/or 1,3 trans-diol functionality of the/said glycoproteins or glycostructures. In particular, the chemical compound which is capable of binding to the/said glycoproteins or glycostructures, preferably to the pyranoid-diol functionality of the/said glycoproteins or glycostructures, more preferably to the 1,2 cis-diol and/or 1,3 trans-diol functionality of the/said glycoproteins or glycostructures, is a boronate or a derivative thereof, a phenylboronic acid or a derivative thereof, an arsenic acid or a derivative thereof, a phenylarsenic acid or a derivative thereof, a metal glycoside chelate, a metal cis-diol chelate, a metal amino acid chelate, a metal alkyl hydroxy acid chelate, preferably a metal methyl hydroxy acid chelate, a metal complex of an amino acid amide, and a metal complex of a tetracycline antibiotic, an N-N-coordinated transition metal complex, preferably selected from the group consisting of a metal complex of ethylendiamine, o-phenylendiamine, phenanthroline, bipyridine, pyridazine, pyrazol, 1,2-diazepine, 1,8-naphthyridine, anthranilic acid amide, oxalic acid diamide, phthalic acid diamide, and a derivative thereof, an O-O-coordinated transition metal complex, preferably selected from the group consisting of a transition metal complex of catechol, a transition metal complex of arene cis-dihydrodiol, and a derivative thereof, a titanium compound or a derivative thereof, a titanium complex or a derivative thereof, or a zirconium compound or a derivative thereof, or a zirconium complex or a derivative thereof. The metals of the metal glycoside chelates, metal cis-diol chelates, metal amino acid chelates, metal alkyl hydroxy acid chelates, metal complexes of amino acid amides, N-N-coordinated transition metal complexes and O-O-coordinated transition metal complexes may be further selected from the divalent and polyvalent d-orbital metals of the first, second and third transition series of the Periodic Table of Elements, from Group IV to Group VIII, including in particular Cu, Mn, Ti, Zr, Zn, V, Cr, Mo, W, Fe, Co, Ni. Suitable Cu (II) complexes of amino acid amides include Cu II-complexes of tryptophanamide, phenylalaninamide, prolinamide and penicillamin. The boronic acid derivatives may comprise an additional electron-withdrawing group resulting from the overall reduced pKa of the boronate molecule. Such molecules include Wulff-type boronates and benzoboroxoles. As used herein, the term "derivative" of a chemical compound refers to a chemical compound that has been chemically modified so that it comprises other chemical groups than the chemical compound from which it is derived.

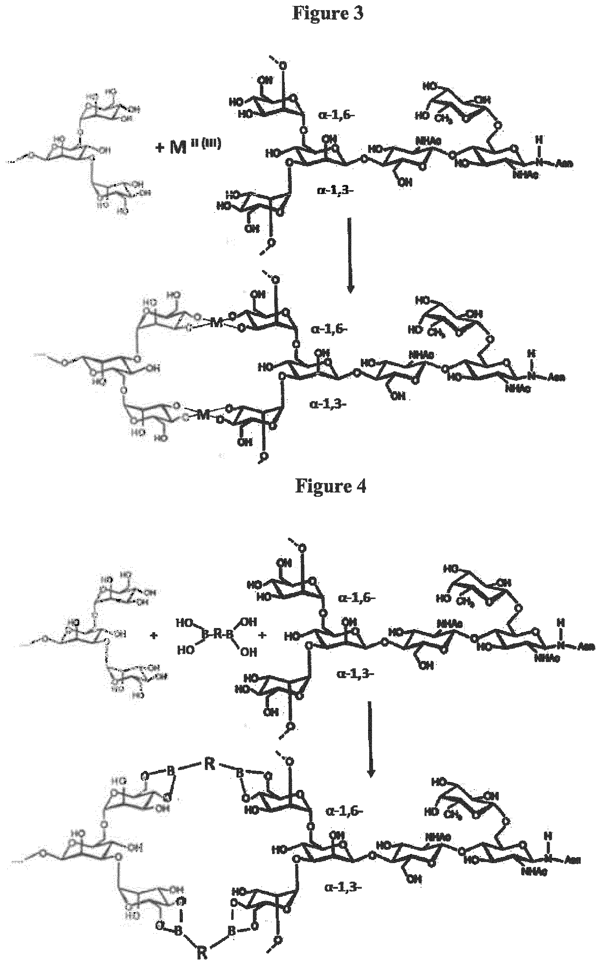

In a further preferred embodiment, the chemical compound which is capable of binding to the/said glycoproteins or glycostructures, preferably to the pyranoid-diol functionality of the/said glycoproteins or glycostructures, more preferably to the 1,2 cis-diol and/or 1,3 trans-diol functionality of the/said glycoproteins or glycostructures, is a multivalent ligand. In the context of the present invention, the term "multivalent ligand" means a ligand which comprises multivalent copies of ligands conjugated to scaffolds allowing the simultaneous binding of multivalent ligands to multiple binding sites. A multivalent ligand having two copies of ligands is, for example, a bivalent ligand, a multivalent ligand having three copies of ligands is, for example, a trivalent ligand, and a multivalent ligand having four copies of ligands is, for example, a tetravalent ligand. In general, the term "multivalency", as used herein, means a key working principle of molecular recognition, based on the notion that individually weak interactions show synergistically increased affinity when cumulatively combined, i.e. binding between two molecules involving more than one binding site occurs with an affinity higher than the sum of all individual binding events. Any of the chemical compounds listed above may form a multivalent ligand. For example, the multivalent ligand may be selected from the group consisting of diboronic acid or a derivative thereof and diphenyldiboronic acid or a derivative thereof. The multivalent ligand may also be a metal complex of diphenyldicatechol or a derivative thereof including pyrocatechol sulfonephthalein (catechol violet). In a particularly preferred embodiment, the multivalent ligand is a complex of a metal, for example copper (II) or cobalt (III), and a glycoside, preferably a α1-3, α1-6 trimannosylglycoside. Such a complex is exemplarily shown in **Figure 3****.** Such complexes may include complexes of alpha-1,3-alpha-1,6-mannotriose and copper (II) or cobalt (III) alone. Such complexes may also include complexes of alpha-1,3-alpha-1,6-mannotriose, copper (II) or cobalt (III), and a chelating agent such as tartrate, diethylamine, or triethyamine. In another particularly preferred embodiment of the method of the first or second aspect of the present invention, the multivalent ligand is a complex of a diboronic acid and a glycoside, preferably a α1-3, α1-6 trimannosylglycoside. Such a complex is exemplarily shown in **Figure 4****.** The diboronic acid may be selected from the group consisting of 2,2'-Bithiophene-5,5'-diboronic acid, 1,4-phenylendiboronic acid (synm. 1,4-benzenediboronic acid), 2-Nitrobenzene-1,4-diboronic acid (synm 2-nitro-1,4-phenylenediboronic acid), 4-4'-biphenyldiboronic acid, 2,5-thiophenediboronic acid, stilbene-4,4-diboronic acid, 4,4'-oxybis(1,4-benzene)diboronic acid, dibenzofuran-4,6-diboronic acid and derivatives thereof.

According to the method of the first or second aspect of the present invention, the chemical compound capable of binding to the glycoproteins or glycostructures on the envelopes of the enveloped viruses is
a boronate, or
a complex of a metal and a glycoside.

The vertebrate cell culture supernatant used in the method of the first or second aspect of the present invention is preferably a mammalian, a fish, an amphibian, a reptilian, or an avian cell culture supernatant. It is particularly preferred that (i) the mammalian cell culture supernatant is a human, hamster, canine, or monkey cell culture supernatant, preferably a Chinese hamster ovary (CHO) cell culture supernatant, (ii) the fish cell culture supernatant is an Ictalurus punctatus (channel catfish) ovary (CCO) cell culture supernatant, (iii) the amphibian cell culture supernatant is a Xenopus laevis kidney cell culture supernatant, the reptilian cell culture supernatant is an Iguana iguana heart (IgH-2) cell culture supernatant, or (iv) the avian cell culture supernatant is a duck cell culture supernatant.

Further described herein but not encompassed by the present invention is a vertebrate cell for producing a glycoprotein or an enveloped virus comprising glycostructures on its envelope, said cell comprises
a polynucleotide sequence comprising a first nucleotide sequence encoding a lactate converting enzyme and a second nucleotide sequence encoding a signal peptide, wherein the signal peptide is capable of directing the lactate converting enzyme to the culture medium,
wherein said cell expresses said enzyme and said signal peptide.

The enzyme which is expressed from the vertebrate cell is preferably an enzyme which is normally not present in the vertebrate cell, i.e. a heterologous or artificial enzyme, e.g. an enzyme from an organism of another kingdom, such as from prokaryotes, e.g. bacteria.

Said cell may be a recombinant cell. Said cell expresses a recombinant lactate converting enzyme.

In a preferred embodiment of the vertebrate cell, the lactate converting enzyme is a (S)-2-hydroxy-acid oxidase or a lactate dehydrogenase. The (S)-2-hydroxy-acid oxidase as lactate converting enzyme is preferably a decarboxylating lactate oxidizing enzyme. It decarboxylates lactate into acetate, carbon dioxide, and water.

In a more preferred embodiment of the vertebrate cell, the (S)-2-hydroxy-acid oxidase is a lactate-2-monooxygenase or a lactate oxidase, most preferably a lactate-2-monooxygenase, e.g. a decarboxylating lactate-2-monooxygenase. With respect to the lactate converting enzyme, it is also referred to the method of the first or second aspect of the present invention.

Preferably, said first and second nucleotide sequences are operably linked to each other. As used herein "operably linked" means that one nucleotide sequence is linked to a second nucleotide sequence in such a way that in-frame expression of a corresponding (fusion) protein can be affected avoiding frame-shifts or stop codons. It is further preferred that the polynucleotide sequence comprises vertebrate specific expression control sequences which allow the expression of said enzyme and said signal peptide in the vertebrate cell. The term "expression control sequences", as used herein, refers to nucleotide sequences which affect the expression of coding sequences in vertebrate cells. Expression control sequences are sequences which control the transcription, e.g. promoters, TATA-boxes, enhancers, codon-usages, post-transcriptional events, e.g. polyadenylation events, and/or translation of nucleotide sequences. In a further preferred embodiment of the vertebrate cell, the polynucleotide sequence is transiently present or stably maintained in the vertebrate cell, either episomally or chromosomally. Thus, the polynucleotide sequence may be stably integrated into the genome of the vertebrate cell.

In another preferred embodiment of the vertebrate cell, the vertebrate cell comprises (i) a nucleotide sequence encoding a glycoprotein, or (ii) an enveloped virus comprising glycostructures on its envelope or a nucleotide sequence encoding an enveloped virus comprising glycostructures on its envelope. The vertebrate cell expresses the glycoprotein or the enveloped virus comprising glycostructures on its envelope. The glycoprotein preferably comprises a signal peptide allowing its secretion into the culture medium. Said signal peptide is expressed in said cell. In this case, the (first) nucleotide sequence encoding a glycoprotein is comprised, together with a second nucleotide sequence encoding a signal peptide, in a polynucleotide sequence, wherein the signal peptide is capable of directing the glycoprotein to the culture medium. Preferably, said first and second nucleotide sequences are operably linked to each other. It is further preferred that the polynucleotide sequence comprises vertebrate specific expression control sequences which allow the expression of said glycoprotein and said signal peptide in the vertebrate cell. Alternatively, the nucleotide sequence encoding an enveloped virus can be comprised in a polynucleotide sequence. The polynucleotide sequence may also comprise vertebrate specific expression control sequences.

The above polynucleotide sequences can be integrated separately into expression vectors, e.g. transient expression vectors or vectors which integrate into the genome of the cell. Alternatively, the above polynucleotide sequences can be comprised on one expression vector. The expression may be driven from one promoter or two promoters. In the former case, it is preferred that the enzyme and the glycoprotein or the enzyme and the enveloped virus comprising glycostructures on its envelope encoded by the polynucleotides are transcribed as a single transcript and are only separated by an IRES. Regarding the preferred embodiments of the glycoprotein or enveloped virus, it is also referred to the method of the first or second aspect of the present invention.

The vertebrate cell is preferably a mammalian, a fish, an amphibian, a reptilian, or an avian cell. It is particularly preferred that (i) the mammalian cell is a human, hamster, canine or monkey cell, preferably a Chinese hamster ovary cell (CHO) cell, (ii) the fish cell is an Ictalurus punctatus (channel catfish) ovary (CCO) cell, (iii) the amphibian cell is a Xenopus laevis kidney cell, the reptilian cell is an Iguana iguana heart (IgH-2) cell, or (iv) the avian cell is a duck cell.

Vertebrate cell culture supernatants from industrial vertebrate producer cell lines contain dominant lactate that competes with the glycoproteins for the binding positions of the ligand in the glycoprotein capture step. Said ligand has an affinity for the pyranoid-diol functionality, particularly 1,2 cis-diol and/or 1,3 trans-diol functionality, of the glycoproteins as well as an affinity for the alpha-hydroxy acid functionality of the lactate. Lactate is the most dominant interfering substance in vertebrate cell culture supernatants that renders direct capture of the glycoproteins impractical. The removal of lactate from the vertebrate cell culture supernatant is, therefore, an immediate prerequisite for enabling direct capture of glycoproteins.

Further described herein but not encompassed by the present invention is a method for purifying glycoproteins or enveloped viruses comprising glycostructures on their envelopes comprising the steps of:
(i) cultivating the vertebrate cell of the third aspect in a culture medium, and
(ii) separating the vertebrate cell from the culture medium and thereby obtaining a lactate-free vertebrate cell culture supernatant comprising glycoproteins or enveloped viruses comprising glycostructures on their envelopes.

The cultivation of the vertebrate cell is preferably performed under conditions that allow the growth of said cell. In particular, the vertebrate cell is cultivated under conditions that allow the production of the lactate converting enzyme and preferably the production of the glycoproteins or enveloped viruses comprising glycostructures on their envelopes. The separation of the vertebrate cell from the culture medium is preferably performed by centrifugation, sedimentation and/or filtration techniques. Other techniques to separate the vertebrate cell from the culture medium in order to achieve the vertebrate cell culture supernatant are known to the skilled person.

In a preferred embodiment, the method further comprises the step of
(iii) applying the lactate-free vertebrate cell culture supernatant to a solid support comprising a chemical compound capable of binding to the glycoproteins or glycostructures on the envelopes of the enveloped viruses.

Preferably, said method additionally comprises the step(s) of:
(iv) washing the solid support with a washing buffer, and/or
(v) releasing the glycoproteins or enveloped viruses comprising glycostructures on their envelopes from the solid support with an elution buffer containing a competitive eluent.

Preferably, steps (iii), (iv), and/or (v) are conducted at a pH between 6 and 8, more preferably at a pH between 6.5 and 7.5, even more preferably at a pH between 6.8 and 7.2, e.g. at a neutral pH (pH 7). Regarding the preferred embodiments of the "chemical compound", "solid support", "washing buffer", "elution buffer", and "competitive eluent", it is referred to the method of the first aspect of the present invention.

In an alternative preferred embodiment, the method further comprises the step of
(iii) precipitating the glycoproteins or enveloped viruses comprising glycostructures on their envelopes from the lactate-free vertebrate cell culture supernatant with a chemical compound capable of binding to said glycoproteins or glycostructures.

Preferably, said method additionally comprises the step(s) of:
(iv) washing the precipitate with a washing buffer, and/or
(v) releasing the glycoproteins or enveloped viruses comprising glycostructures on their envelopes from the precipitate with an elution buffer (dissociation buffer) containing a competitive eluent (competitive dissociating reactant).

Preferably, steps (iii), (iv), and/or (v) are conducted at a pH between 6 and 8, more preferably at a pH between 6.5 and 7.5, even more preferably at a pH between 6.8 and 7.2, e.g. at a neutral pH (pH 7). Regarding the preferred embodiments of the "chemical compound", "washing buffer", "elution buffer (dissociation buffer)", and "competitive eluent (competitive dissociating reactant)", it is referred to the method of the second aspect of the present invention.

Further described herein but not encompassed by the present invention is a method for purifying glycoproteins or enveloped viruses comprising glycostructures on their envelopes from a vertebrate cell culture supernatant comprising the steps of:
(i) contacting a vertebrate cell culture supernatant comprising glycoproteins or enveloped viruses comprising glycostructures on their envelopes and lactate with a lactate converting enzyme, and
(ii) applying the vertebrate cell culture supernatant to a solid support comprising a chemical compound capable of binding to the glycoproteins or glycostructures on the envelopes of the enveloped viruses.

All preferred embodiments mentioned with respect to the method of the first aspect of the present invention also apply to the method described above.

Also described herein but not encompassed by the present invention is a method for purifying glycoproteins or enveloped viruses comprising glycostructures on their envelopes from a vertebrate cell culture supernatant comprising the steps of:
(i) contacting a vertebrate cell culture supernatant comprising glycoproteins or enveloped viruses comprising glycostructures on their envelopes and lactate with a lactate converting enzyme, and
(ii) precipitating the glycoproteins or enveloped viruses comprising glycostructures on their envelopes from the vertebrate cell culture supernatant with a chemical compound capable of binding to said glycoproteins or glycostructures.

All preferred embodiments mentioned with respect to the method of the second aspect of the present invention also apply to the method described above.

Some specific preferred embodiments regarding the above methods are mentioned below: In a preferred embodiment, the method further described above further comprises the step(s) of:
(iii) washing the solid support with a washing buffer, and/or
(iv) releasing the glycoproteins or enveloped viruses comprising glycostructures on their envelopes from the solid support with an elution buffer containing a competitive eluent.

In a preferred embodiment, the method also described above further comprises the step(s) of:
(iii) washing the precipitate with a washing buffer, and/or
(iv) releasing the glycoproteins or enveloped viruses comprising glycostructures on their envelopes from the precipitate with an elution buffer (dissociation buffer) containing a competitive eluent (competitive dissociating reactant).

The competitive eluent (competitive dissociating reactant) used in step (iv) of the above described methods is preferably selected from the group consisting of furanoid cis diol sugars or glycosides, pyranoid 1,2,3 triol sugars or glycosides, sugar alcohols, preferably sorbitol, adonitol, mannitol, xylitol, dulcitol, pentaerythritol, or erythritol, alpha hydroxy carbonic acids, preferably glycolate, lactate, malate, tartrate, citrate, alpha-hydroxy caprylate, salicylate or glucuronate, and hydroxylamino compounds, preferably triethanolamin or Tris-hydroxymethyl-aminomethan (TRIS).

Preferably, steps (ii), (iii), and/or (iv) of the above described methods are conducted at a pH between 6 and 8, more preferably at a pH between 6.5 and 7.5, even more preferably at a pH between 6.8 and 7.2, e.g. at a neutral pH (pH 7).

In a preferred embodiment of the above described methods, the lactate converting enzyme is a (S)-2-hydroxy-acid oxidase, particularly a decarboxylating (S)-2-hydroxy-acid oxidase, or a lactate dehydrogenase. Preferably, the (S)-2-hydroxy-acid oxidase is a lactate-2-monooxygenase or a lactate oxidase, preferably a lactate-2-monooxygenase, e.g. a decarboxylating lactate-2-monooxygenase. Regarding other preferred embodiments with respect to the lactate converting enzyme, it is referred to the method of the first or second aspect of the present invention.

In a preferred embodiment of the above described methods, the chemical compound mentioned in step (ii) is capable of binding to the pyranoid-diol functionality of the/said glycoproteins or glycostructures, more preferably to the 1,2 cis-diol and/or 1,3 trans-diol functionality of said glycoproteins or glycostructures. It is preferred that said compound is a boronate or a derivative thereof, a phenylboronic acid or a derivative thereof, an arsenic acid or a derivative thereof, a phenylarsenic acid or a derivative thereof, a metal glycoside chelate, a metal cis-diol chelate, a metal amino acid chelate, a metal alkyl hydroxy acid chelate, preferably a metal methyl hydroxy acid chelate, a metal complex of an amino acid amide, and a metal complex of a tetracycline antibiotic, an N-N-coordinated transition metal complex, preferably selected from the group consisting of a metal complex of ethylendiamine, o-phenylendiamine, phenanthroline, bipyridine, pyridazine, pyrazol, 1,2-diazepine, 1,8-naphthyridine, anthranilic acid amide, oxalic acid diamide, phthalic acid diamide, and a derivative thereof, an O-O-coordinated transition metal complex, preferably selected from the group consisting of a boron transition metal complex, a transition metal complex of catechol, a transition metal complex of arene cis-dihydrodiol, and a derivative thereof, a titanium compound or a derivative thereof, a titanium complex or a derivative thereof, or a zirconium compound or a derivative thereof, or a zirconium complex or a derivative thereof.

It is also preferred that said chemical compound is a multivalent ligand. Any of the chemical compounds listed above may form a multivalent ligand. For example, the multivalent ligand may be selected from the group consisting of diboronic acid or a derivative thereof and diphenyldiboronic acid or a derivative thereof. The multivalent ligand may also be a metal complex of diphenyldicatechol or a derivative thereof including pyrocatechol sulfonephthalein (catechol violet). In a preferred embodiment of the above described methods, the multivalent ligand is a complex of a metal and a glycoside, preferably a α1-3, α1-6 trimannosylglycoside. In another preferred embodiment of the above described methods, the multivalent ligand is a complex of a diboronic acid and a glycoside, preferably a α1-3, α1-6 trimannosylglycoside.

In a further preferred embodiment of the above described methods, the vertebrate cell culture supernatant is a mammalian, a fish, an amphibian, a reptilian, or an avian cell culture supernatant. Regarding other preferred embodiments with respect to the vertebrate cell culture supernatant, it is referred to the first or second aspect of the present invention. Regarding the preferred embodiments of the glycoproteins or enveloped viruses, it is also referred to the first or second aspect of the present invention.

Additionally described herein but not encompassed by the present invention is a multivalent ligand which is a complex of (i) a metal and a glycoside, preferably a α1-3, α1-6 trimannosylglycoside, or (ii) a diboronic acid and a glycoside, preferably a α1-3, α1-6 trimannosylglycoside. The metal may be copper (II) or cobalt (III). Preferably, the multivalent ligand is a complex as shown in **Figure 3** or a complex as shown in **Figure 4****.**

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

The following figures and examples are merely illustrative of the present invention and should not be construed to limit the scope of the invention as indicated by the appended claims in any way.

### BRIEF DESCRIPTION OF THE FIGURES

**Figure 1****:** Integration of a lactate conversion step into the early downstream unit operations. An enzyme bioreactor (EZMBR) equipped with a lactate oxidase is fitted between the unit operations for cell separation and the glyco-affinity capture step (black hatched column). CS= cell separation module.
**Figure 2****:** Core-fucosylated biantennary, bigalactosylated G2F-structure of an N-linked glycan. The alpha-1,6-core fucosylated trimannosyl-chitobiose core structure is contained within the dotted rectangle, the fucose residue is shown in grey, glycosidic bonds of the trimannosyl structure are indicated. The vicinal pyranosidic diol-structures of the trimannosyl-structure are highlighted by dotted grey circles. Above: Vicinal Mannopyranosyl-trans equatorial 3,4-diol structures unfavourable for boronate binding but suited for metal chelation. Below: Vicinal Mannopyranosyl-cis-4,6-diol structures suited for both boronate binding and metal chelation.
**Figure 3****:** Schematic representation of multivalent complex formation between alpha-1,3-alpha-1,6-mannotriose metal chelates and N-linked glycans.
**Figure 4****:** Schematic representation of multivalent complex formation between alpha-1,3-alpha-1,6-mannotriose, diboronic acids and N-linked glycans.

### EXAMPLES

### Example 1: Immobilization of lactate oxidase on Immobeads 150

4U of lyophilized lactate oxidase (Sigma-Aldrich UK Sigma, L0638) are resuspended in 10 µl 40 mM glutaraldehyde solution and incubated for two hours at 4°C, before adding 100 mg of dry beads (Immobead 150 (Sigma, ChiralVision, 94129)) and incubating for 48 hours at 4°C. After incubation excess glutaraldehyde is quenched by adding 10 mM NaBH3CN (Aldrich 156159) in 10 mM potassium phosphate buffer. For each 100 mg Immobeads, 370 µl 10 mM potassium phosphate buffer are added and the beads are then incubated at 4°C for 24 hours followed by two hours incubation with 1500 µl 10 mM NaBH₃CN to give a final concentration of 8 mM.

### Example 2: Immobilization of S-lactate-2-monooxygenase on Sepharose 4B (agarose gel)

5 ml sepharose 4B are washed with water (20 x 5 ml), 0.06 M phosphate buffer pH=5.6 (20 x 5 ml), water (20 x 5 ml), 50 mM sodium carbonate buffer, pH=9.6 (20 x 5 ml). The sepharose 4B is then suspended in 10 ml 50 mM carbonate buffer, pH=9.6 and 1 ml 10 mM divinylsulphone. The mixture is then shaken for 80 min. The activated sepharose 4B is washed with water (20 x 5 ml), 0.06 M phosphate buffer, pH=5.6 (20 x 5 ml), water (20 x 5 ml), and 50 mM carbonate buffer pH=9.6 (20 x 5 ml). The activated sepharose 4 B is resuspended in 5 ml 50 mM carbonate buffer and then 0.5 ml of dissolved lactate-2-monooxygenase (from Mycobacterium smegmatis, e.g. SEQ ID NO: 1) in 50 mM carbonate buffer, pH 9.6 are added and the mixture is agitated at RT for 18 hours. The solid immobilized sepharose 4B-lactate-monooxygenase is separated by filtration, suspended in 10 ml 50 mM glycine buffer, pH=9.6 and agitated at RT for another 2 hours. After the incubation, the immobilized sepharose 4B-lactate-monooxygenase is washed with water (20 x 5 ml), 0.06 M phosphate buffer (20 x 5 ml), water (20 x 5 ml), and 50 mM TRIS buffer, pH 7.4 (20 x 5 ml).

### Example 3: Conversion of lactate from cell culture supernatant with the immobilized lactate oxidase in a batch process

10 mg of the immobilized lactate oxidase obtained in Example 1 are suspended in 100 ml end-of-process (day 12/14) spent medium from CHO producer clones (lactate conc.: 300-2500 mg/l) inside a closed vial and incubated at 37°C. 100 µl samples are taken at intervals of 1, 3, 5 and 7 hours and analyzed for lactate content. After completion of lactate removal, beads are sedimented and the supernatant is direct applied onto a glyco-affinity capture column (as described in Example 4).

### Example 4: Direct Purification of glycoproteins from lactate-depleted spent medium

Lactate-deleted spent medium from Example 3 containing the glycosylated Fc-fusionprotein Etanercept is loaded at 4°C onto a ProSepPB column (Millipore Corporation, Billerica, Mass., USA) that has been equilibrated with 0.1 M sodium borate + 0.15 M NaCl, pH 8.5. After loading, the lactate-depleted spent medium onto the column unbound material is washed out with 7 column volumes of 0.1 M sodium borate buffer + 0.15 M NaCl, pH 8.5. Finally, the captured Etanercept glycoprotein is eluted at RT by binary gradient step elution with an elution buffer consisting of 200 mM sodium tartrate, 50 mM Tris, pH 7.6.

### Example 5: Generation of a CHO-K1 host cell line expressing an Fc-fusionprotein and a lacate-2-monooxygenase

### 5.1 Generation of a stable CHO-K1 cell line expressing an Fc-fusionprotein

CHO-K1 cells are transfected with an expression vector containing an expression cassette comprising a nucleotide sequence encoding a therapeutic Fc-fusionprotein. The cell line is maintained in serum-free medium under antibiotic and auxotrophic selection until the transgene has stably integrated into the host cell line's genome. These CHO-Fc cells are then maintained under serum-free medium conditions.

### 5.2 Gene Optimization and Synthesis

The amino acid sequence for the lactate-2-monooxygenase (Mycobacterium smegmatis, 394 amino acids) (GenBank Accession No. AAA60429.1, SEQ ID NO: 1) is reverse translated and the resulting nucleotide sequence optimized by knockout of cryptic splice sites and RNA destabilizing sequence elements, optimization for increased RNA stability and adaptation of codon usage to match the requirements of CHO cells (*Cricetulus griseus*).

### 5.3 Construction of the Lactate-2-monooxygenase Expression Plasmid

The synthesized lactate-2-monooxygenase-construct is cut with restriction endonucleases and dephoshorylated with calf intestinal phosphatase. The digested and dephosphorylated insert is ligated into a pre-digested bicistronic expression vector which allows coordinated co-expression of lactate-2-monooxygenase and green fluorescent protein from a bicistronic message (gfp). The expression plasmid is equipped with a Neomycin resistance gene allowing for direct selection of cells that have stably integrated the bicistronic expression cassette. General procedures for constructing expression plasmids are described in Sambrook, J., E. F. Fritsch and T. Maniatis: Cloning I/II/III, A Laboratory Manual New York/Cold Spring Harbor Laboratory Press, 1989, Second Edition.

### 5.4 Transfection of CHO-K1 cells

CHO-Fc cells stably expressing the therapeutic Fc-fusionprotein are stably transfected with the lactate-2-monooxygenase transgene by electroporation according to the manufacturer's instructions (MicroPorator, PEQLAB Biotech, Germany). 24 h after electroporation, transfectants are selected in alpha-MEM containing the antibiotic G418. The G418-resistant clones are then isolated by limiting dilution cloning, i.e. they are resuspended in this selective medium and seeded into 96 well plates at dilutions where the likelihood of obtaining a colony from a single cell is greater 95% based on poisson statistics. To assure monoclonality, cells grown within the 96 wells are isolated and again seeded into 96 well plates at limiting dilution. After these two rounds of single cell cloning, a couple of the isolated single cell clones are expanded into larger volumes. Afterwards, they are adapted to growth in suspension. Using the described electroporation protocol a transformation efficiency of approximately 2000 per 2×10⁶ electroporated cells is achieved as assessable from gfp-fluorescence distribution in the culture dishes.

### 5.5 Cultivation of CHO-Fc-Lactate-2-monooxigenase cells

The CHO-Fc-Lactate-2-monooxigenase cells are inoculated into shaker tubes at 2×10⁵ cells/ml in a serum-free growth medium. The shaker tubes are incubated at 180 rpm, 37° C, 7.5% pCO2. The culture is stopped after 7 days when the cells still show a vitality >80%. Viable cell density is measured with an automatic cell counter using trypan blue exclusion. Cells are then pelleted by centrifugation for 10 at 5000 rpm and the supernatant is transferred into a separate vial and analyzed for residual lactate concentration.

### REFERENCES

[1] Myöhänen TA, Bouriotis V, Dean PD. Affinity chromatography of yeast alpha-glucosidase using ligand-mediated chromatography on immobilized phenylboronic acids. Biochem J. 1981 Sep 1;197(3):683-8.
[2] Abraham EC, Perry RE, Stallings M Application of affinity chromatography for separation and quantitation of glycosylated hemoglobins. J Lab Clin Med.1983 Aug;102(2):187-97.)
[3] Liu X-C and Scouten, WH Boronate Affinity Chromatography, in Methods in Molecular Biology, vol. 147: Affinity Chromatography: Methods and ProtocolsEdited by: P. Bailon, G. K. Ehrlich, W.-J. Fung, and W. Berthold © Humana Press Inc., Totowa, NJ
[4] Singhal, R. P., Ramamurthy, B., Govindraj, N., and Sarwar, Y. (1991) New ligands for boronate affinity chromatography. Synthesis and properties. J. Chromatogr. 543, 17-38.
[5] Liu, X.-C. and Scouten, W. H. (1994) New ligands for boronate affinity chromatography. J. Chromatogr. A 687, 61-69.
[6] Liu XC1, Scouten WH. Studies on oriented and reversible immobilization of glycoprotein using novel boronate affinity gel. J Mol Recognit. 1996 Sep-Dec;9(5-6):462-7.
[7] Li H, Liu Y, Liu J, Liu Z. A Wulff-type boronate for boronate affinity capture of cis-diol compounds at medium acidic pH condition. Chem Commun (Camb). 2011 Jul 28;47(28):8169-71.
[8] Li H, Wang H, Liu Y, Liu Z. A benzoboroxole-functionalized monolithic column for the selective enrichment and separation of cis-diol containing biomolecules. Chem Commun (Camb). 2012 Apr 28;48(34):4115-7.
[9] Bull SD1, Davidson MG, van den Elsen JM, Fossey JS, Jenkins AT, Jiang YB, Kubo Y, Marken F, Sakurai K, Zhao J, James TD. Exploiting the reversible covalent bonding of boronic acids: recognition, sensing, and assembly. Acc Chem Res. 2013 Feb 19;46(2):312-26. doi: 10.1021/ar300130w. Epub 2012 Nov 14.
[10] Friedman S , Pace B , Pizer R Complexation of phenylboronic acid with lactic acid. Stability constant and reaction kinetics J. Am. Chem. Soc., 1974, 96 (17), pp 5381-5384
[11] Pizer R , Selzer R The boric acid/lactic acid system. Equilibria and reaction mechanism Inorg. Chem., 1984, 23 (19), pp 3023-3026
[12] Bromba C, Carriea P, Chui JKW, Fyles TM, Phenyl boronic acid complexes of diols and hydroxyacids Supramolecular Chemistry Volume 21, Issue 1-2, 2009
[13] Warburg O (1956). "On the origin of cancer cells". Science 123 (3191): 309-314.

## Claims

1. A method for purifying glycoproteins or enveloped viruses comprising glycostructures on their envelopes from a vertebrate cell culture supernatant comprising the steps of:
(i) providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) applying the vertebrate cell culture supernatant to a solid support comprising a chemical compound capable of binding to the glycoproteins or glycostructures on the envelopes of the enveloped viruses,
wherein the chemical compound capable of binding to the glycoproteins or glycostructures on the envelopes of the enveloped viruses is a boronate, or
a complex of a metal and a glycoside, and
wherein step (ii) is conducted at a pH between 6 and 8.

2. A method for purifying glycoproteins or enveloped viruses comprising glycostructures on their envelopes from a vertebrate cell culture supernatant comprising the steps of:
(i) providing a vertebrate cell culture supernatant comprising glycoproteins or enveloped viruses comprising glycostructures on their envelopes, wherein said vertebrate cell culture supernatant is free of lactate or has a lactate concentration below 2.0 g/l, and
(ii) precipitating the glycoproteins or enveloped viruses comprising glycostructures on their envelopes from the vertebrate cell culture supernatant with a chemical compound capable of binding to said glycoproteins or glycostructures, wherein the chemical compound capable of binding to the glycoproteins or glycostructures on the envelopes of the enveloped viruses is a boronate, or
a complex of a metal and a glycoside, and
wherein step (ii) is conducted at a pH between 6 and 8.

3. The method of claims 1 or 2, wherein the vertebrate cell culture supernatant provided in step (i) is generated by contacting a vertebrate cell culture supernatant comprising glycoproteins or enveloped viruses comprising glycostructures on their envelopes and lactate with a lactate converting enzyme.

4. The method of claims 1 or 3, wherein the method further comprises the step(s) of:
(iii) washing the solid support with a washing buffer, and/or
(iv) releasing the glycoproteins or enveloped viruses comprising glycostructures on their envelopes from the solid support with an elution buffer containing a competitive eluent.

5. The method of claims 2 or 3, wherein the method further comprises the step(s) of:
(iii) washing the precipitate with a washing buffer, and/or
(iv) releasing the glycoproteins or enveloped viruses comprising glycostructures on their envelopes from the precipitate with an elution buffer containing a competitive eluent.

6. The method of claims 4 or 5, wherein steps (iii) and/or (iv) are conducted at a pH between 6 and 8, preferably at a pH between 6.5 and 7.5, more preferably at a pH between 6.8 and 7.2, most preferably at pH 7.

7. The method of any one of claims 3 to 6, wherein the lactate converting enzyme is a (S)-2-hydroxy-acid oxidase or a lactate dehydrogenase.

8. The method of claim 7, wherein the (S)-2-hydroxy-acid oxidase is a lactate-2-monooxygenase or a lactate oxidase, preferably a lactate-2-monooxygenase.

9. The method of any one of claims 1 to 8, wherein the chemical compound is capable of binding to the pyranoid-diol functionality of the/said glycoproteins or glycostructures.

10. The method of claim 1, wherein the complex of a metal and a glycoside is a α1-3, α1-6 trimannosylglycoside.

11. The method of any one of claims 4 to 10, wherein the competitive eluent is selected from the group consisting of furanoid cis diol sugars or glycosides, pyranoid 1,2,3 triol sugars or glycosides, sugar alcohols, preferably sorbitol, adonitol, mannitol, xylitol, dulcitol, pentaerythritol, or erythritol, alpha hydroxy carbonic acids, preferably glycolate, lactate, malate, tartrate, citrate, alpha-hydroxy caprylate, salicylate or glucuronate, and hydroxylamino compounds, preferably triethanolamin or Tris-hydroxymethyl-aminomethan (TRIS).

## Patentansprüche

1. Verfahren zum Aufreinigen von Glykoproteinen oder Hüllviren, die Glykostrukturen an ihren Hüllen umfassen, aus einem Wirbeltierzellkultur-Überstand, umfassend die folgenden Schritte:
(i) Bereitstellen eines Wirbeltierzellkultur-Überstands, umfassend Glykoproteine oder Hüllviren, die Glykostrukturen an ihren Hüllen umfassen, wobei der Wirbeltierzellkultur-Überstand frei von Laktat ist oder eine Laktatkonzentration unter 2,0 g/l aufweist, und
(ii) Aufbringen des Wirbeltierzellkultur-Überstands auf einen festen Träger, umfassend eine chemische Verbindung, die in der Lage ist, an die Glykoproteine oder Glykostrukturen an den Hüllen der Hüllviren zu binden,
wobei die chemische Verbindung, die in der Lage ist, an die Glykoproteine oder Glykostrukturen an den Hüllen der Hüllviren zu binden,
ein Boronat, oder
ein Komplex aus einem Metall und einem Glykosid ist, und
wobei Schritt (ii) bei einem pH zwischen 6 und 8 ausgeführt wird.

2. Verfahren zum Aufreinigen von Glykoproteinen oder Hüllviren, die Glykostrukturen an ihren Hüllen umfassen, aus einem Wirbeltierzellkultur-Überstand, umfassend die folgenden Schritte:
(i) Bereitstellen eines Wirbeltierzellkultur-Überstands, umfassend Glykoproteine oder Hüllviren, die Glykostrukturen an ihren Hüllen umfassen, wobei der Wirbeltierzellkultur-Überstand frei von Laktat ist oder eine Laktatkonzentration unter 2,0 g/l aufweist, und
(ii) Präzipitieren der Glykoproteine oder Hüllviren, die Glykostrukturen an ihren Hüllen umfassen, aus dem Wirbeltierzellkultur-Überstand mit einer chemischen Verbindung, die in der Lage ist, an die Glykoproteine oder Glykostrukturen zu binden,
wobei die chemische Verbindung, die in der Lage ist, an die Glykoproteine oder Glykostrukturen an den Hüllen der Hüllviren zu binden,
ein Boronat, oder
ein Komplex aus einem Metall und einem Glykosid ist, und
wobei Schritt (ii) bei einem pH zwischen 6 und 8 ausgeführt wird.

3. Verfahren nach den Ansprüchen 1 oder 2, wobei der in Schritt (i) bereitgestellte Wirbeltierzellkultur-Überstand erzeugt wird, in dem ein Wirbeltierzellkultur-Überstand umfassend Glykoproteine oder Hüllviren, die Glykostrukturen an ihren Hüllen umfassen, und Laktat mit einem Enzym, das Laktat umwandelt, in Kontakt gebracht wird.

4. Verfahren nach den Ansprüchen 1 oder 3, wobei das Verfahren ferner den/die folgenden Schritt(e) umfasst:
(iii) Waschen des festen Trägers mit einem Waschpuffer, und/oder
(iv) Freisetzen der Glykoproteine oder Hüllviren, die Glykostrukturen an ihren Hüllen umfassen, von dem festen Träger mit einem Elutionspuffer, der einen kompetitiven Eluenten enthält.

5. Verfahren nach den Ansprüchen 2 oder 3, wobei das Verfahren ferner den/die folgenden Schritt(e) umfasst:
(iii) Waschen des Präzipitats mit einem Waschpuffer, und/oder
(iv) Freisetzen der Glykoproteine oder Hüllviren, die Glykostrukturen an ihren Hüllen umfassen, von dem Präzipitat mit einem Elutionspuffer, der einen kompetitiven Eluenten enthält.

6. Verfahren nach den Ansprüchen 4 oder 5, wobei die Schritte (iii) und/oder (iv) bei einem pH zwischen 6 und 8, vorzugsweise bei einem pH zwischen 6,5 und 7,5, bevorzugter bei einem pH zwischen 6,8 und 7,2, am bevorzugtesten bei einem pH von 7, ausgeführt werden.

7. Verfahren nach einem der Ansprüche 3 bis 6, wobei das Enzym, das Laktat umwandelt, eine (S)-2-Hydroxysäure-Oxidase oder eine Laktatdehydrogenase ist.

8. Verfahren nach Anspruch 7, wobei die (S)-2-Hydroxysäure-Oxidase eine Laktat-2-monooxygenase oder eine Laktatoxidase, vorzugsweise eine Laktat-2-monooxygenase, ist.

9. Verfahren nach einem der Ansprüche 1 bis 8, wobei die chemische Verbindung in der Lage ist, an die Pyranoid-diol-Funktionalität der Glykoproteine oder Glykostrukturen zu binden.

10. Verfahren nach Anspruch 1, wobei der Komplex aus einem Metall und einem Glykosid ein α1-3, α1- 6 Trimannosylglykosid ist.

11. Verfahren nach einem der Ansprüche 4 bis 10, wobei der kompetitive Eluent ausgewählt ist aus der Gruppe bestehend aus Furanoid-cis-diol-Zuckern oder -Glykosiden, Pyranoid-1,2,3-Triol-Zuckern oder -Glykosiden, Zuckeralkoholen, vorzugsweise Sorbitol, Adonitol, Mannitol, Xylitol, Dulcitol, Pentaerythritol oder Erythritol, alpha-Hydroxycarbonsäuren, vorzugsweise Glykolat, Laktat, Malat, Tartrat, Citrat, alpha-Hydroxycaprylat, Salicylat oder Glukuronat, und Hydroxylamino-Verbindungen, vorzugsweise Triethanolamin oder Tris-hydroxymethyl-aminomethan (TRIS).

## Revendications

1. Méthode de purification de glycoprotéines ou de virus enveloppés comprenant des glycostructures sur leurs enveloppes provenant d'un surnageant de culture de cellules de vertébrés, comprenant les étapes consistant à :
(i) fournir un surnageant de culture de cellules de vertébrés comprenant des glycoprotéines ou des virus enveloppés comprenant des glycostructures sur leurs enveloppes, ledit surnageant de culture de cellules de vertébrés étant exempt de lactate ou ayant une concentration de lactate inférieure à 2,0 g/l, et
(ii) appliquer le surnageant de culture de cellules de vertébrés sur un support solide comprenant un composé chimique capable de liaison aux glycoprotéines ou glycostructures sur les enveloppes des virus enveloppés,
le composé chimique capable de liaison aux glycoprotéines ou glycostructures sur les enveloppes des virus enveloppés étant
un boronate, ou
un complexe constitué d'un métal et d'un glycoside, et
où l'étape (ii) est effectuée à un pH compris entre 6 et 8.

2. Méthode de purification de glycoprotéines ou de virus enveloppés comprenant des glycostructures sur leurs enveloppes provenant d'un surnageant de culture de cellules de vertébrés comprenant les étapes consistant à :
(i) fournir un surnageant de culture de cellules de vertébrés comprenant des glycoprotéines ou des virus enveloppés comprenant des glycostructures sur leurs enveloppe, ledit surnageant de culture de cellules de vertébrés étant exempt de lactate ou ayant une concentration de lactate inférieure à 2,0 g/l, et
(ii) précipiter les glycoprotéines ou les virus enveloppés comprenant des glycostructures sur leurs enveloppes provenant du surnageant de culture de cellules de vertébrés avec un composé chimique capable de liaison auxdites glycoprotéines ou glycostructures,
le composé chimique capable de liaison aux glycoprotéines ou glycostructures sur les enveloppes des virus enveloppés étant
un boronate, ou
un complexe constitué d'un métal et d'un glycoside, et
où l'étape (ii) est effectuée à un pH compris entre 6 et 8.

3. Procédé de la revendication 1 ou 2, dans lequel le surnageant de culture de cellules de vertébrés fourni à l'étape (i) est généré par la mise en contact d'un surnageant de culture de cellules de vertébrés comprenant des glycoprotéines ou des virus enveloppés comprenant des glycostructures sur leurs enveloppes et du lactate avec une enzyme de conversion en lactate.

4. Procédé de la revendication 1 ou 3, le procédé comprenant en outre la ou les étape(s) consistant à :
(iii) laver le support solide avec un tampon de lavage, et/ou
(iv) libérer les glycoprotéines ou virus enveloppés, comprenant des glycostructures sur leurs enveloppes, du support solide avec un tampon d'élution contenant un éluant compétitif.

5. Procédé de la revendication 2 ou 3, le procédé comprenant en outre la ou les étape(s) consistant à :
(iii) laver le précipité avec un tampon de lavage, et/ou
(iv) libérer les glycoprotéines ou virus enveloppés, comprenant des glycostructures sur leurs enveloppes, du précipité avec un tampon d'élution contenant un éluant compétitif

6. Procédé de la revendication 4 ou 5, dans lequel les étapes (iii) et/ou (iv) sont effectuées à un pH compris entre 6 et 8, de préférence à un pH compris entre 6,5 et 7,5, plus préférablement à un pH compris entre 6,8 et 7,2, plus préférablement encore au pH 7.

7. Procédé de l'une quelconque des revendications 3 à 6, dans lequel l'enzyme de conversion en lactate est une (S)-2-hydroxy-acide oxydase ou un lactate déhydrogénase.

8. Procédé de la revendication 7, dans lequel la (S)-2-hydroxy-acide oxydase est un lactate-2- monooxygénase ou un lactate oxydase, de préférence un lactate-2-monooxygénase.

9. Procédé de l'une quelconque des revendications 1 à 8, dans lequel le composé chimique est capable de liaison à la fonctionnalité pyranoid-diol desdites glycoprotéines ou glycostructures.

10. Procédé de la revendication 1, dans lequel le complexe constitué d'un métal et d'un glycoside est un α1-3, α1- 6 trimannosylglycoside.

11. Procédé selon l'une quelconque des revendications 4 à 10, dans lequel l'éluant compétitif est choisi dans le groupe constitué de sucres furanosiques cis diol ou glycosides, sucres pyranosiques 1,2,3 triol ou glycosides, alcools de sucre, de préférence sorbitol, adonitol, mannitol, xylitol, dulcitol, pentaérythritol, ou érythritol, acides carboxyliques alpha-hydroxy, de préférence glycolate, lactate, malate, tartrate, citrate, caprylate alpha-hydroxy, salicylate ou glucuronate, et composé hydroxylamino, de préférence triéthanolamine ou Tris-hydroxyméthyl-aminométhane (TRIS).
